# FASCICULE DE BREVET EUROPEEN

(11) **EP 2 276 474 B1**
(45) Date de publication et mention de la délivrance du brevet: **06.03.2019**
(21) Numéro de dépôt: 09745961.4
(22) Date de dépôt: 17.04.2009
(51) Int. Cl.: A61K 9/26, A61K 9/52, A61K 9/58, A61K 9/62

(54) **FORME ORALE SOLIDE DOTEE D'UN DOUBLE PROFIL DE LIBERATION COMPRENANT DES MULTIPARTICULES**
FESTE ORALE MULTIPARTIKELHALTIGE DARREICHUNGSFORM MIT ZWEIFACHEM FREISETZUNGSPROFIL
SOLID ORAL FORM WITH DUAL RELEASE PROFILE, CONTAINING MULTIPARTICULATES

(30) Priorité: 18.04.2008 FR 0852627; 18.04.2008 US 71250 P
(43) Date de publication de la demande: 26.01.2011
(73) Titulaire: Flamel Ireland Limited, Dublin 15 (IE)
(72) Inventeur: GUIMBERTEAU, Florence, F-33450 Montussan (FR); DAVIAUD, Anne-Sophie, F-69230 Saint Genis Laval (FR)
(74) Mandataire: Nony
(86) Numéro de dépôt international: PCT/FR2009/050719
(87) Numéro de publication internationale: WO 2009/138642

(56) Documents cités:
- EP-A- 0 438 249
- EP-A- 1 258 241
- WO-A-02/13794
- US-A- 5 458 888
- US-A- 5 800 836
- US-A1- 2001 009 678
- US-A1- 2003 161 874
- US-A1- 2004 018 234
- US-A1- 2004 043 996
- US-A1- 2005 118 268
- US-A1- 2006 263 429

## Description

La présente invention vise à proposer une forme solide ou encore comprimé, destinée à l'administration par voie orale, renfermant au moins un actif formulé à l'état de microparticules, lesdites microparticules sous forme libre et ladite forme solide finale les contenant étant dotées du même profil spécifique de libération modifiée.

La présente invention vise également un procédé utile pour la préparation d'une telle forme solide.

L'art antérieur a montré qu'il était intéressant de disposer de formes pharmaceutiques orales multiparticulaires. Ces systèmes microparticulaires sont constitués d'un grand nombre de microcapsules ou de microparticules de diamètre généralement inférieur à 2000 µm. Ces systèmes sont avantageux à plusieurs titres.

Tout d'abord, la dose d'actif(s) à administrer s'y trouve répartie en un grand nombre de microparticules, typiquement 10 000 pour une dose de 500 mg, et présente de ce fait une faible sensibilité à la variabilité de la vidange gastrique et un risque quasi-nul de mise en contact des tissus avec une dose élevée en actif(s).

Par ailleurs, les systèmes multiparticulaires autorisent la mise en oeuvre, au sein d'une unique unité de dose telle qu'une gélule par exemple, d'un mélange de microparticules de profils de libération modifiée différents, permettant ainsi de réaliser des profils de libération présentant plusieurs vagues de libération ou assurant par un réglage adéquat des différentes proportions, un niveau de concentration plasmatique en actif(s) constant.

A titre illustratif de ces formes de libération modifiée à l'état multiparticulaire, on peut notamment citer celles décrites dans les documents US 2002/0192285, US 6 238 703, US 2002/0192285, US 2005/0118268 et US 5,800,836 et notamment celles décrites dans la demande WO 03/030878.

On peut encore citer le document US 2006/263429, qui décrit des comprimés à libération prolongée de morphine, comprenant notamment des granulés de morphine enrobés par un mélange d'Eudragit® L100 et d'éthylcellulose.

Le document US 2004/0018234 décrit la préparation de comprimés comprenant des granulés contenant un antibiotique (Amoxicilline ou Clarithromycine) enrobés par une couche d'enrobage formée à partir de la pulvérisation sur les granulés d'une composition contenant de l'Eudragit® L 30D-55 et de l'Eudragit® NE 30D.

Quant au document US 2001/0009678, il décrit la préparation de comprimés comprenant des poudres contenant un actif pharmaceutique (le Lansoprazole), enrobées par pulvérisation d'une solution de revêtement comprenant de l'Eudragit® L30D-55 et de l'Eudragit® NE30D.

On peut encore citer le document US 5,458,888, qui décrit une forme pharmaceutique à libération contrôlée d'actif, pouvant être compressés sous forme de comprimé, comprenant une phase interne contenant l'actif sous forme de particules enrobées et une phase externe comprenant un polyéthylène glycol.

US 2003/161874 décrit quant à lui des particules comprenant un enrobage comprenant un copolymère d'acide méthacrylique (environ 35 % en poids de l'enrobage), de l'éthylcellulose, de l'acétylcritrate de tributyle et du talc. Enfin, US 2005/118268 décrit des particules enrobées présentant un enrobage comprenant de l'HPMC phtalate et de l'éthylcellulose.

En particulier, la demande WO 03/030878 décrit plus particulièrement des formes pharmaceutiques orales multiparticulaires, permettant une libération de l'actif qu'elles contiennent selon un double mécanisme de libération modifiée, le premier étant conditionné par le temps et le second étant conditionné par le pH. Plus précisément, ce processus de libération peut être schématisé par un enchaînement de trois phases distinctes : une première phase dite de latence suivie par une deuxième phase dite de libération contrôlée, et qui se manifestent toutes deux au contact d'un milieu acide représentatif du milieu stomacal, suivie par une troisième phase dite de libération accélérée voire immédiate et qui se manifeste au contact d'un milieu neutre représentatif du milieu intestinal.

Ce système multiparticulaire permet ainsi une libération de l'actif modifiée, retardée et prolongée, et dont les différentes séquences sont déclenchées selon deux mécanismes distincts respectivement activés par le temps et par le pH. Le passage de la première phase à la deuxième phase est déclenché par un temps de contact avec le milieu acide représentatif du milieu stomacal, alors que le passage de la deuxième phase à la troisième phase est déclenché par le changement de pH rencontré lorsque les microparticules quittent l'estomac pour entrer dans l'intestin.

Ce type particulier de profil de libération modifiée est particulièrement intéressant dans les cas suivants :
- lorsqu'on cherche une libération retardée, soit pour adapter la libération du principe actif à un cycle chronobiologique tout en maintenant un horaire de prise compatible avec la vie courante, soit pour retarder la libération d'un principe actif par rapport à un autre au sein d'une combinaison ; et
- lorsque le principe actif considéré est fortement métabolisé par le foie sans que les métabolites soient actifs. Une libération modifiée sous forme de plusieurs pics décalés permet dans ce cas de minimiser le métabolisme hépatique et de préserver la biodisponibilité tout en prolongeant la durée d'action du principe actif.

Dans tous ces cas, les formulations présentant le profil de libération spécifique en 3 phases sont supérieures en termes de variabilité aux formulations communément appelées entériques et généralement utilisées pour obtenir une libération retardée.

Les formulations entériques conventionnelles ne présentent en effet que 2 phases : une phase de non libération ou encore de latence en milieu acide représentatif du milieu stomacal et une phase de libération immédiate en milieu neutre représentatif du milieu intestinal. Dans le cas de ces formes entériques classiques, la libération du principe actif est déclenchée par le changement de pH lié au passage de la forme de l'estomac dans l'intestin. Or ce passage est extrêmement variable d'un individu à l'autre, et même d'un moment à l'autre au sein du même individu. Il n'est pas rare qu'une forme orale soit retenue dans l'estomac, bien plus longtemps que prévu, jusqu'à 18 h par exemple. Ainsi, pour un produit administré toutes les 24 heures, si le premier comprimé est retenu 18 h et si le comprimé suivant passe dans l'intestin bien plus rapidement, le patient ne sera pas traité le premier jour, mais recevra l'équivalent de 2 doses le second jour. Cette variabilité peut avoir des conséquences négatives si le principe actif présente un index thérapeutique faible, c'est-à-dire si un taux plasmatique élevé de cet actif est associé à des effets secondaires graves.

Les formulations présentant un profil de libération spécifique en 3 phases évitent ce problème et permettent d'accéder à des profils de libération retardée et prolongée avec une variabilité faible et acceptable, même pour des principes actifs à index thérapeutique faible.

Elles se présentent généralement sous la forme de microparticules ou microcapsules dont le coeur, contenant l'actif ou un mélange d'actifs, est recouvert d'un enrobage dont la composition et/ou l'épaisseur sont précisément ajustées pour contrôler la libération de cet actif selon deux mécanismes distincts, selon que le coeur enrobé est localisé dans l'estomac ou le petit intestin, l'un étant conditionné par la durée du séjour dans un milieu aqueux acide et l'autre par le pH du milieu contenant les microparticules.

Par exemple, l'enrobage des microparticules décrites dans WO 03/03878 est formé d'un matériau comprenant au moins un polymère hydrophile porteur de groupements ionisés à pH neutre, à l'image par exemple d'un copolymère d'acide (méth)acrylique et de (méth)acrylate d' alkyle et d'au moins un composé hydrophobe à l'image d'une cire hydrogénée végétale. De telles microparticules donnent totale satisfaction en termes de profil de libération modifiée, lorsqu'elles sont formulées en un système galénique non compressé à l'image d'une poudre ou d'une gélule.

Malheureusement, la formulation de ce type de microparticules sous une forme solide orale compressée à l'image d'un comprimé, s'avère préjudiciable au profil de libération modifiée. En particulier, le temps de latence initial est généralement perdu sous l'effet de la libération accélérée de l'actif d'une partie au moins des microparticules dont l'enrobage a été brisé par la force de compression, appliquée lors de la formulation du comprimé.

Or, parmi l'ensemble des formes solides, les formes solides compactées ou encore cohésives, à l'image des comprimés, sont avantageuses à plusieurs titres.

Contrairement aux poudres, elles ne requièrent pas une pré-dilution dans un milieu aqueux et donc peuvent être ingérées instantanément par le patient avec en outre une totale garantie au niveau du dosage de l'actif reçu par le patient. Par ailleurs, leur production industrielle est nettement moins contraignante pour le formulateur comparativement aux gélules ou capsules. De plus, par rapport à celles-ci, les formes solides de type comprimé possèdent une meilleure résistance mécanique. Elles ne sont pas friables tout en étant compatibles avec une fragmentation en plusieurs parties si nécessaire en termes de posologie (comprimés sécables).

Il demeure donc un besoin d'une formulation solide présentée sous forme de comprimé et constituée d'un grand nombre de microparticules dotées d'un profil de libération spécifique en trois phases pour l'actif véhiculé au sein de cette formulation.

En particulier il demeure un besoin d'une forme solide comprimée constituée de microparticules à libération modifiée en trois phases telle que la forme comprimée finale présente le même profil de libération modifiée en trois phases que les microparticules à libération modifiée qu'elle contient, considérées sous forme libre.

En particulier, il demeure un besoin d'une formulation solide apte à procurer un profil de libération modifiée en trois phases, résultant d'un double mécanisme de libération, le premier étant conditionné par le temps, dans la mesure où la libération de l'actif est déclenchée après un temps de séjour déterminé de la formulation solide au niveau de l'estomac et, le second étant conditionné par le pH, dans la mesure où la libération de l'actif est accélérée dès la mise en contact de la formulation solide avec le milieu contenu dans le petit intestin.

De manière inattendue, les inventeurs ont constaté qu'il est possible de disposer de formes orales, solides, formées par compression, et néanmoins aptes à garantir un tel profil de libération d'actif(s) modifiée en trois phases, sous réserve de disperser, au sein de ces formes solides, l'actif sous la forme de microparticules dotées d'un enrobage spécifique.

Plus précisément, la présente invention décrit une forme solide destinée à l'administration par voie orale d'au moins un actif et apte à garantir un double mécanisme de libération dudit actif, le premier étant conditionné par le temps et le second étant conditionné par le pH, caractérisée en ce que ledit actif y est présent sous la forme d'un système microparticulaire dont les microparticules possèdent un coeur formé en tout ou partie dudit actif et enrobé d'au moins une couche conditionnant ledit profil de libération modifiée dudit actif et formée d'un matériau composé d'au moins :
- 10 à 75 % en poids, et en particulier 25 à 75 % en poids par rapport au poids total dudit enrobage d'au moins un polymère A insoluble dans les fluides gastro-intestinaux,
- 25 à 90 % en poids, et en particulier 25 à 75 % en poids par rapport au poids total dudit enrobage d'au moins un polymère B possédant une valeur de pH de solubilisation variant dans la plage de pH de 5 à 7 et
- 0 à 25 % en poids par rapport au poids total dudit enrobage d'au moins un plastifiant,
lesdits polymères A et B étant présents dans un rapport pondéral polymère(s) B/polymère(s) A au moins égal à 0,25.

L'expression « double mécanisme de libération dudit actif, le premier étant conditionné par le temps et le second étant conditionné par le pH » peut également être désignée, de manière simplifiée, sous les termes « double mécanisme de libération en termes de temps et de pH ».

Au sens de l'invention, on désigne de façon générale par « forme solide orale » des comprimés destinés à l'administration par voie orale.

L'expression « double mécanisme de libération » traduit le fait que les microparticules présentent deux mécanismes distincts de libération de l'actif que l'on peut également schématiser sous la forme d'un profil de libération à trois phases :
- un premier mécanisme de libération différée dans le temps au contact d'un milieu acide. Ce mécanisme de libération peut être découpé en une première phase de latence suivie d'une deuxième phase de libération contrôlée. En d'autres termes, les formes solides orales selon l'invention possèdent une aptitude à initier le relargage de l'actif qu'elles contiennent en milieu aqueux acide, qu'au terme d'une mise en contact avec ce milieu d'au moins 30 minutes.
- un seconde mécanisme de libération accélérée voire immédiate, au contact d'un milieu aqueux neutre. Ce second mécanisme de libération peut être schématisé par une troisième phase de libération.

De ce fait, la forme solide considérée selon l'invention est apte, d'une part, à libérer de façon prolongée l'actif qu'elle contient après un temps de latence, conditionné par un temps de séjour donné au niveau de l'estomac et, d'autre part, à déclencher une libération accélérée de l'actif lors de l'entrée de la forme solide dans l'intestin où elle est confrontée à une augmentation de pH. Ces deux mécanismes de libération de l'actif ou des actifs formulés dans la forme solide selon l'invention sont assurés en séquence.

Au sens de la présente invention, la valeur de pH de solubilisation du polymère B est une valeur de pH du milieu physiologique ou du milieu in vitro modèle en-deçà de laquelle le polymère est insoluble et au-delà de laquelle ce même polymère B est soluble.

Pour des raisons évidentes, cette valeur de pH est spécifique à un polymère donné et directement liée à ses caractéristiques physico-chimiques intrinsèques, telles que sa nature chimique et sa longueur de chaîne.

Au sens de la présente invention, une forme solide est une forme solide dotée d'une résistance mécanique à la rupture. Elle est aussi avantageusement non déformable.

Au regard de ces spécificités, elle se distingue des formes galéniques également qualifiées de « solide », telles que par exemple les gélules et les poudres.

Avantageusement, une forme solide selon l'invention se présente sous une forme matricielle dans laquelle sont dispersées les microparticules contenant l'actif ou le mélange d'actifs à véhiculer.

Plus précisément, elles sont obtenues par compression des différents composés et/ou matériaux entrant dans leur composition.

Selon un mode de réalisation préféré, la forme solide selon l'invention est une forme de type comprimé.

Ainsi, un premier objet de l'invention est un comprimé, destiné à l'administration par voie orale d'au moins un actif et apte à garantir un double mécanisme de libération dudit actif, le premier étant conditionné par le temps et le second étant conditionné par le pH, dans lequel ledit actif y est présent sous la forme d'un système microparticulaire dont les microparticules possèdent un coeur formé en tout ou partie dudit actif et enrobé d'au moins une couche conditionnant ledit profil de libération dudit actif et formée d'un matériau composé d'au moins :
- 30 à 50% en poids par rapport au poids total dudit enrobage d'au moins un polymère A insoluble dans les fluides gastro-intestinaux, choisi parmi l'éthylcellulose, l'acétate butyrate de cellulose, l'acétate de cellulose, les copolymères d'ammonio (méth)acrylate de type « A » ou de type « B », les esters d'acides poly(méth)acryliques, et leurs mélanges ;
- 30 à 60% en poids par rapport au poids total dudit enrobage d'au moins un polymère B possédant une valeur de pH de solubilisation comprise dans la plage de pH de 5 à 7, choisi parmi :
   ∘ le(s) copolymère(s) d'acide méthacrylique et de méthacrylate de méthyle,
   ∘ le(s) copolymère(s) d'acide méthacrylique et d'acrylate d'éthyle,
   ∘ les dérivés cellulosiques tels que l'acétate phtalate de cellulose, l'acétate succinate de cellulose, l'acétate trimellilate de cellulose, le phtalate d'hydroxypropylméthylcellulose, l'acétate succinate d'hydroxypropyl méthylcellulose,
   ∘ la gomme shellac,
   ∘ le polyvinyle d'acétate phtalate, et
   ∘ leurs mélanges, et
ladite couche d'enrobage des microparticules étant composée d'une unique couche formée dudit matériau ;
lesdites microparticules étant dispersées au sein du comprimé,
lesdites microparticules étant aptes à libérer ledit actif selon un profil de libération modifiée en trois phases caractérisé par :
- au contact d'un milieu dont le pH est à une valeur inférieure à celle du pH de solubilisation du polymère B, un profil de libération retardée et prolongée avec un temps de latence donné et compris entre 0,5 et 12 heures, en particulier entre 0,5 et 8 heures, voire entre 1 et 5 heures et selon un temps de demie libération t_{1/2} compris entre 0,75 et 24 heures, notamment entre 0,75 et 12 heures, voire entre 0,75 et 8 heures, en particulier entre 1 et 5 heures, temps au terme duquel la moitié de l'actif contenu est libérée ;
- au contact d'un milieu dont le pH est à une valeur supérieure à celle du pH de solubilisation du polymère B, une libération de l'actif sans temps de latence et avec un t_{1/2} compris entre 0,1 et 2 heures ;
la cinétique de libération de l'actif pouvant être déterminée par des tests de dissolution *in vitro*, effectués dans un appareil à palettes USP type II avec une vitesse de rotation des palettes de 75 rpm, par spectrométrie UV, d'une part, dans un milieu 0,1 N HCl et, d'autre part, dans un milieu phosphate de potassium 0,05 M à pH 6,8.

Selon un mode de réalisation préféré, la forme solide possède une dureté variant de 50 à 500 N.

Selon une variante de réalisation, une forme solide conforme à l'invention peut contenir au moins deux types de microparticules, lesdits types différant entre eux au moins par la nature de l'actif qu'ils contiennent et/ou par la composition et/ou l'épaisseur de l'enrobage formant leurs particules respectives.

Selon une autre variante de réalisation, la composition solide selon l'invention peut comprendre au moins deux types de microparticules, se distinguant l'un de l'autre par des profils de libération distincts.

Selon encore une autre variante de réalisation, une forme solide selon l'invention peut contenir outre des particules possédant un double mécanisme de libération tel que défini précédemment, des particules dotées d'un profil de libération immédiate du ou des actifs qu'elles contiennent.

Selon encore un autre de ses aspects, la présente invention concerne un procédé pour l'obtention d'une forme solide conforme à l'invention, telle que défini ci-après.

Enfin, selon un autre de ses aspects, la présente invention concerne des microparticules spécifiques telles que définies ci-après.

### Forme solide

Comme précisé précédemment, une forme solide selon l'invention, de type comprimé, est avantageusement produite par compression. Elle peut bien entendu subir par ailleurs des traitements complémentaires, notamment tels que définis ci-après.

Compte-tenu de ce mode de préparation, elle possède une résistance significative à la rupture. Par exemple, pour un comprimé rond de diamètre 12 mm, cette dureté peut varier de 50 à 500 N, en particulier de 60 à 200 N.

Cette dureté peut être mesurée selon le protocole décrit dans la Pharmacopée Européenne 6ème édition chapitre 2.9.8..

De manière inattendue, cette cohésion mécanique ne s'avère pas préjudiciable par ailleurs à la manifestation, par les microparticules dispersées dans ladite composition solide, du profil de libération modifiée spécifique en trois phases du ou des actif(s) qu'elles véhiculent.

Les microparticules, lorsqu'elles sont libérées de la matrice formant la forme solide selon l'invention, généralement par délitage de celle-ci au contact d'un milieu aqueux, demeurent aptes grâce à la composition spécifique de leur enrobage à libérer le principe actif selon un profil de libération modifiée spécifique en trois phases, tel que décrit précédemment, au sein du tractus gastro-intestinal.

Plus précisément, lorsque ces microparticules sont placées dans un milieu dont le pH est à une valeur inférieure à celle du pH de solubilisation du polymère B formant lesdites particules, il est observé un profil de libération retardée et prolongée avec un temps de latence donné et compris entre 0,5 et 12 heures, en particulier entre 0,5 et 8 heures, voire entre 1 et 5 heures et selon un temps de demie libération t_{1/2} compris entre 0,75 et 24 heures, notamment entre 0,75 et 12 heures, voire entre 0,75 et 8 heures, en particulier entre 1 et 5 heures, temps au terme duquel la moitié de l'actif contenu est libéré.

En revanche, lorsque ces mêmes microparticules, ayant séjourné au préalable dans l'estomac ou dans un milieu assimilable c'est-à-dire de pH inférieur au pH de solubilisation du polymère B, sont mises en présence avec un milieu dont le pH est à une valeur supérieure à celle du pH de solubilisation du polymère B formant lesdites particules, il est observé une libération de l'actif sans temps de latence et avec un t_{1/2} compris entre 0,1 et 10 heures, en particulier entre 0,1 et 5 heures, notamment entre 0,1 et 2 heures.

Le temps de latence correspond au temps en-dessous duquel les microparticules libèrent moins de 20 % de leur dose en actif(s).

### Système microparticulaire

L'invention comprend des microparticules dont la composition et l'architecture sont ajustées pour conférer précisément le profil de libération modifiée recherché pour l'actif ou mélange d'actifs qu'elles contiennent.

Plus précisément, les microparticules considérées selon l'invention sont structurellement organisées en un coeur, formé en tout ou partie d'au moins un actif ou d'un mélange d'actifs, et enrobé ou pelliculé par un enrobage.

Ce coeur peut être :
- de l'actif brut (pur), et/ou
- un granulé matriciel contenant l'actif ou mélange d'actif(s) mélangé à différents autres ingrédients, et/ou
- un granulé obtenu par l'application d'une couche formée en tout ou partie de l'actif sur une particule support, par exemple constituée de cellulose ou de sucre.

Dans le cas d'un granulé matriciel, la matrice peut contenir l'actif et éventuellement d'autres excipients physiologiquement acceptables, tels que des agents liants, des tensioactifs, des désintégrants, des charges, des agents contrôlant ou modifiant le pH (tampons).

Dans le cas de l'utilisation d'une particule support, celle-ci peut être composée de saccharose et/ou de dextrose et/ou de lactose, et/ou de mélange saccharose/amidon. Elle peut également être une microsphère de cellulose ou toute autre particule d'excipient physiologiquement acceptable. Avantageusement, la particule support a un diamètre moyen inférieur à 1500 µm et de préférence compris entre 20 et 1000 µm, de préférence entre 50 et 1000 µm, en particulier entre 50 et 800 µm, voire entre 50 et 600 µm. La couche active peut éventuellement comporter, outre le (ou les) actif(s), un ou plusieurs excipients physiologiquement acceptables, tels que des agents liants, des tensioactifs, des désintégrants, des charges, des agents contrôlant ou modifiant le pH (tampons).

Selon une variante de réalisation particulière, le coeur formant les microparticules est un granulé obtenu par l'application d'une couche formée en tout ou partie de l'actif sur une particule support telle que définie ci-dessus.

Dans le cas de la présente invention, l'enrobage possède une composition ajustée pour procurer le profil de libération spécifique de l'actif ou mélange d'actifs associé, c'est-à-dire en trois phases déclenchées par un double mécanisme de libération activé par le temps et le pH.

Plus précisément, l'enrobage est formé d'un matériau composite tel que défini précédemment, obtenu par mélange de :
- au moins un polymère A insoluble dans les liquides du tube digestif ;
- au moins un second polymère B possédant une valeur de pH de solubilisation comprise dans la plage de pH de 5 à 7 ;
- et éventuellement au moins un agent plastifiant et/ou autres excipients conventionnels.

### Polymère A

Des polymères insolubles dans les liquides du tube digestif ou encore les fluides gastro-intestinaux sont par exemple :
- les dérivés non hydrosolubles de la cellulose,
- les dérivés non hydrosolubles de (co)polymères (méth)acryliques,
- et leurs mélanges.

Le polymère A selon l'invention est choisi parmi l'éthylcellulose, par exemple ceux commercialisés sous la dénomination Ethocel®, l'acétate butyrate de cellulose, l'acétate de cellulose, les copolymères d'ammonio (méth)acrylate (copolymère d'acrylate d'éthyle, de méthacrylate de méthyle et de méthacrylate de triméthylammonio éthyle) de type « A » ou de type « B » notamment ceux commercialisés sous les dénominations Eudragit® RL et Eudragit® RS, les esters d'acides poly(méth)acryliques, notamment ceux commercialisés sous la dénomination Eudragit® NE et leurs mélanges.

Conviennent tout particulièrement à l'invention l'éthylcellulose, l'acétate butyrate de cellulose et les copolymères d'ammonio (méth)acrylate notamment ceux commercialisés sous la dénomination Eudragit RS® et Eudragit RL®.

L'enrobage des microparticules décrites contient de 10 % à 75 %, et peut contenir de préférence de 15 % à 60 %, plus préférentiellement de 20 % à 55 %, notamment de 25 % à 55 %, et plus particulièrement encore de 30 à 50 % de polymère(s) A par rapport à son poids total.

Selon une variante décrite, l'enrobage des particules contient de 35 % à 65 %, de préférence de 40 % à 60 % en poids de polymère(s) A par rapport à son poids total.

Comme indiqué précédemment, l'enrobage des microparticules selon l'invention contient de 30 à 50 % en poids de polymère(s) A par rapport à son poids total.

### Polymère B

Les polymères (B) selon l'invention sont choisis parmi :
- le(s) copolymère(s) d'acide méthacrylique et de méthacrylate de méthyle,
- le(s) copolymère(s) d'acide méthacrylique et d'acrylate d'éthyle,
- les dérivés cellulosiques tels que :
   ∘ l'acétate phtalate de cellulose (CAP),
   ∘ l'acétate succinate de cellulose (CAS),
   ∘ l'acétate trimellitate de cellulose (CAT),
   ∘ le phtalate d'hydroxypropylméthylcellulose (ou hypromellose phtalate) (HPMCP),
   ∘ l'acétate succinate d'hydroxypropylméthylcellulose (ou hypromellose acetate succinate) (HPMCAS),
- la gomme shellac,
- le polyvinyle d'acétate phtalate (PVAP),
- et leurs mélanges.

Selon un mode préféré de l'invention, ce polymère B est choisi parmi le(s) copolymère(s) d'acide méthacrylique et de méthacrylate de méthyle, le(s) copolymère(s) d'acide méthacrylique et d'acrylate d'éthyle et leurs mélanges.

Comme précisé précédemment, le polymère B considéré selon l'invention possède un profil de solubilité différent selon qu'il est confronté à une valeur de pH supérieure ou inférieure à sa valeur de pH de solubilisation.

Au sens de l'invention, le polymère B est généralement insoluble à une valeur de pH inférieure à sa valeur de pH de solubilisation et en revanche soluble à une valeur de pH supérieure à sa valeur de pH de solubilisation.

Par exemple, il peut s'agir d'un polymère dont la valeur de pH de solubilisation est de :
- 5,0 à l'image par exemple du phtalate d'hydroxypropylméthylcellulose et notamment celui commercialisé sous la dénomination HP-50 par Shin-Etsu,
- 5,5 à l'image par exemple du phtalate d'hydroxypropylméthylcellulose et notamment celui commercialisé sous la dénomination HP-55 par Shin-Etsu ou du copolymère d'acide méthacrylique et d'acrylate d'éthyle 1 :1 et notamment celui commercialisé sous la dénomination Eudragit L100-55 de Evonik,
- 6,0 à l'image par exemple d'un copolymère d'acide méthacrylique et de méthacrylate de méthyle 1 :1 et notamment celui commercialisé sous la dénomination Eudragit L100 de Evonik,
- 7,0 comme par exemple un copolymère d'acide méthacrylique et de méthacrylate de méthyle 1 :2 et notamment celui commercialisé sous la dénomination Eudragit S100 de Evonik.

L'ensemble de ces polymères est soluble à une valeur de pH supérieure à son pH de solubilisation.

L'enrobage des microparticules décrites peut être composé d'au moins 25 à 90 %, en particulier de 30 à 80 %, plus particulièrement de 30 à 75 %, notamment de 35 à 70 %, en particulier de 35 à 65 %, voire de 40 à 60 % en poids de polymère(s) B par rapport à son poids total.

L'enrobage selon l'invention est composé de 30 à 60 % en poids, en particulier de 40 à 60 % en poids, de polymère(s) B par rapport à son poids total.

Avantageusement, l'enrobage est formé à partir d'un mélange des deux catégories de polymères A et B dans un rapport pondéral polymère(s) B/polymère(s) A supérieur à 0,25, en particulier supérieur ou égal à 0,3, en particulier supérieur ou égal à 0,4, notamment supérieur ou égal à 0,5, voire supérieur ou égal à 0,75.

Selon une autre variante de réalisation, le rapport polymère(s) B/polymère(s) A est en outre inférieur à 8, notamment inférieur à 4, voire inférieur à 2 et plus particulièrement inférieur à 1,5.

A titre représentatif des mélanges polymères A et B convenant tout particulièrement à l'invention, peuvent notamment être cités les mélanges d'éthylcellulose, d'acétate butyrate de cellulose ou de copolymère d'ammonio (méth)acrylate de type A ou B avec au moins un copolymère d'acide méthacrylique et d'acrylate d'éthyle ou un copolymère d'acide méthacrylique et de méthacrylate de méthyle ou un de leurs mélanges.

Ainsi, selon un mode de réalisation particulier, les particules selon l'invention peuvent être avantageusement formées d'au moins un couple polymère B/polymère A choisi parmi les couples suivants :
1. copolymère d'acide méthacrylique et d'acrylate d'éthyle, 1:1 / éthylcellulose,
2. copolymère d'acide méthacrylique et de méthacrylate de méthyle 1:2 / éthylcellulose,
3. mélange de copolymère d'acide méthacrylique et d'acrylate d'éthyle 1:1 et de copolymère d'acide méthacrylique et de méthacrylate de méthyle, 1:2 / éthylcellulose,
4. copolymère d'acide méthacrylique et d'acrylate d'éthyle 1:1 / acétate butyrate de cellulose,
5. copolymère d'acide méthacrylique et de méthacrylate de méthyle 1:2 / acétate butyrate de cellulose,
6. mélange de copolymère d'acide méthacrylique et d'acrylate d'éthyle 1:1 et de copolymère d'acide méthacrylique et de méthacrylate de méthyle 1:2 / acétate butyrate de cellulose,
7. copolymère d'acide méthacrylique et d'acrylate d'éthyle 1:1 / copolymère d'ammonio (méth)acrylate de type « A » ou type « B »,
8. copolymère d'acide méthacrylique et de méthacrylate de méthyle 1:2 / copolymère d'ammonio (méth)acrylate de type « A » ou type « B »,
9. mélange de copolymère d'acide méthacrylique et d'acrylate d'éthyle 1:1 et de copolymère d'acide méthacrylique et de méthacrylate de méthyle 1:2 / copolymère d'ammonio (méth)acrylate de type « A » ou type « B ».

La présente invention décrit des microparticules possédant un coeur formé en tout ou partie d'au moins un actif, ledit coeur étant enrobé d'au moins une couche déterminant un double mécanisme de libération, le premier étant conditionné par le temps et le second étant conditionné par le pH dudit actif et formée d'un matériau composé d'au moins :
- 10 à 75 % en poids, et en particulier 25 à 75 % en poids, en particulier de 25 % à 60 % et, plus préférentiellement encore, de 25 % à 55 %, et plus particulièrement encore de 30 à 50 % par rapport au poids total dudit enrobage d'au moins un polymère A insoluble dans les fluides gastro-intestinaux, choisi parmi l'éthylcellulose, l'acétate butyrate de cellulose, un copolymère d'ammonio (méth)acrylate de type « A » ou de type « B », les esters d'acides poly(méth)acrylique et leurs mélanges, et
- 25 à 90 % en poids, et en particulier 25 à 75 % en poids, en particulier de 30 à 75 %, notamment de 35 à 70 %, voire de 40 à 60 % en poids par rapport au poids total dudit enrobage d'au moins un polymère B possédant une valeur de pH de solubilisation comprise dans la plage de pH variant de 5 à 7 et choisi parmi un copolymère d'acide méthacrylique et de méthacrylate de méthyle, un copolymère d'acide méthacrylique et d'acrylate d'éthyle et leurs mélanges.

En particulier, un autre objet de l'invention concerne des microparticules possédant un coeur formé en tout ou partie d'au moins un actif, ledit coeur étant enrobé d'au moins une couche conditionnant un double mécanisme de libération dudit actif, le premier étant conditionné par le temps et le second étant conditionné par le pH,
ladite couche d'enrobage étant formée d'un matériau composé d'au moins :
- 30 à 50 % par rapport au poids total dudit enrobage d'au moins un polymère A insoluble dans les fluides gastro-intestinaux et choisi parmi l'éthylcellulose, l'acétate butyrate de cellulose, un copolymère d'ammonio (méth)acrylate de type « A » ou de type « B », les esters d'acides poly(méth)acrylique, et leurs mélanges, et
- 40 à 60 % en poids par rapport au poids total dudit enrobage d'au moins un polymère B possédant une valeur de pH de solubilisation comprise dans la plage de pH variant de 5 à 7 et choisi parmi un copolymère d'acide méthacrylique et de méthacrylate de méthyle, un copolymère d'acide méthacrylique et d'acrylate d'éthyle et leurs mélanges ;
ladite couche d'enrobage des microparticules étant composée d'une unique couche formée dudit matériau,
lesdites microparticules étant aptes à libérer ledit actif selon un profil de libération modifiée en trois phases caractérisé par :
- au contact d'un milieu dont le pH est à une valeur inférieure à celle du pH de solubilisation du polymère B, un profil de libération retardée et prolongée avec un temps de latence donné et compris entre 0,5 et 12 heures, en particulier entre 0,5 et 8 heures, voire entre 1 et 5 heures et selon un temps de demie libération t_{1/2} compris entre 0,75 et 24 heures, notamment entre 0,75 et 12 heures, voire entre 0,75 et 8 heures, en particulier entre 1 et 5 heures, temps au terme duquel la moitié de l'actif contenu est libérée ;
- au contact d'un milieu dont le pH est à une valeur supérieure à celle du pH de solubilisation du polymère B, une libération de l'actif sans temps de latence et avec un t_{1/2} compris entre 0,1 et 2 heures ;
la cinétique de libération dudit actif pouvant être déterminée par des tests de dissolution in vitro, effectués dans un appareil à palettes USP type II avec une vitesse de rotation des palettes de 75 rpm, par spectrométrie UV, d'une part, dans un milieu 0,1 N HCl et, d'autre part, dans un milieu phosphate de potassium 0,05 M à pH 6,8.

Avantageusement, l'enrobage peut être formé d'un couple polymère B/polymère A choisi parmi les couples précités.

Outre les deux types de polymères précités, l'enrobage des particules selon l'invention peut comprendre au moins un agent plastifiant.

### Agent plastifiant

Cet agent plastifiant peut être notamment choisi parmi :
- le glycérol et ses esters, et de préférence parmi les glycérides acétylés, glycéryl-mono-stéarate, glycéryl-triacétate, glycéryl-tributyrate,
- les phtalates, et de préférence parmi les dibutylphtalate, diéthylphtalate, diméthylphtalate, dioctylphtalate,
- les citrates, et de préférence parmi les acétyltributylcitrate, acétyltriéthylcitrate, tributylcitrate, triéthylcitrate,
- les sébaçates, et de préférence parmi les diéthylsébaçate, dibutylsébaçate,
- les adipates,
- les azélates,
- les benzoates,
- le chlorobutanol,
- les polyéthylènes glycols,
- les huiles végétales,
- les fumarates, de préférence le diéthylfumarate,
- les malates, de préférence le diéthylmalate,
- les oxalates, de préférence le diéthyloxalate,
- les succinates ; de préférence le dibutylsuccinate,
- les butyrates,
- les esters de l'alcool cétylique,
- les malonates, de préférence le diéthylmalonate,
- l'huile de ricin,
- et leurs mélanges.

En particulier, l'enrobage peut comprendre moins de 25 % en poids, de préférence, de 1 % à 20 % en poids, et, plus préférentiellement encore, de 5 % à 20 %, en particulier de 5 % à 15 %, et plus préférentiellement environ 10 % en poids d'agent(s) plastifiant(s) par rapport à son poids total.

L'invention décrit des particules dont l'enrobage peut être formé d'au moins
- 20 à 60 %, en particulier de 30 à 60 % en poids d'au moins un polymère A choisi parmi l'éthylcellulose, l'acétate butyrate de cellulose, un copolymère d'ammonio (méth)acrylate de type « A » ou de type « B » ou un de leurs mélanges,
- de 30 à 70 %, en particulier de 40 à 70 % en poids d'au moins un polymère B choisi parmi un copolymère d'acide méthacrylique et de méthacrylate de méthyle, notamment un copolymère d'acide méthacrylique et de méthacrylate de méthyle 1:1 ou un copolymère d'acide méthacrylique et de méthacrylate de méthyle 1:2; un copolymère d'acide méthacrylique et d'acrylate d'éthyle, notamment un copolymère d'acide méthacrylique et d'acrylate d'éthyle 1:1 ou un copolymère d'acide méthacrylique et d'acrylate d'éthyle 1:2, et leurs mélanges
- et d'environ 10 % en poids d'au moins un agent plastifiant tel que par exemple le triéthylcitrate.

A titre illustratif et non limitatif de ces particules, on peut notamment citer celles dont l'enrobage possède l'une des compositions suivantes.
- 30 à 60 % d'éthylcellulose
   40 à 70 % de copolymère d'acide méthacrylique et d'acrylate d'éthyle 1 :1
   10 % de triéthylcitrate
- 30 à 60 % d'éthylcellulose
   40 à 70 % de copolymère d'acide méthacrylique et de méthacrylate de méthyle 1:2
   10 % de triéthylcitrate
- 30 à 60 % d'éthylcellulose
   40 à 70 % d'un mélange de copolymère d'acide méthacrylique et d'acrylate d'éthyle 1 :1 et de copolymère d'acide méthacrylique et de méthacrylate de méthyle 1:2
   10 % de triéthylcitrate
- 30 à 60 % d'acetate butyrate de cellulose
   40 à 70 % de copolymère d'acide méthacrylique et d'acrylate d'éthyle 1 :1
   10 % de triéthylcitrate
- 30 à 60 % d'acétate butyrate de cellulose
   40 à 70 % de copolymère d'acide méthacrylique et de méthacrylate de méthyle 1 :2
   10 % de triéthylcitrate
- 30 à 60 % d'acetate butyrate de cellulose
   40 à 70 % d'un mélange de copolymère d'acide méthacrylique et d'acrylate d'éthyle 1 :1 et de copolymère d'acide méthacrylique et de méthacrylate de méthyle 1:2
   10 % de triéthylcitrate
- 30 à 60 % de copolymère d'ammonio (méth)acrylate de type « A » ou type « B » 40 à 70 % de copolymère d'acide méthacrylique et d'acrylate d'éthyle 1 :1
   10 % de triéthylcitrate
- 30 à 60 % de copolymère d'ammonio (méth)acrylate de type « A » ou type « B » 40 à 70 % de copolymère d'acide méthacrylique et de méthacrylate de méthyle 1:2
   10 % de triéthylcitrate et
- 30 à 60 % de copolymère d'ammonio (méth)acrylate de type « A » ou type « B » 40 à 70 % d'un mélange de copolymère d'acide méthacrylique et d'acrylate d'éthyle 1 :1 et de copolymère d'acide méthacrylique et de méthacrylate de méthyle 1:2
   10 % de triéthylcitrate.

Bien entendu, l'enrobage peut comprendre divers autres adjuvants additionnels utilisés classiquement dans le domaine de l'enrobage. Il peut s'agir, par exemple, de pigments, de colorants, de charges, d'agents anti-mousse, d'agents tensioactifs etc.

Selon un mode de réalisation particulier de l'invention, l'enrobage ne contient pas de principe actif.

Selon un autre mode de réalisation de l'invention, l'enrobage est dénué de composé soluble à une valeur de pH allant de 1 à 4.

L'enrobage peut être mono- ou multi-couche. Selon l'invention, il est composé d'une unique couche formée à partir du matériau composite défini précédemment.

L'enrobage des microparticules, qu'elles soient à l'état libre ou dispersées au sein d'une composition solide selon l'invention, possède avantageusement le même aspect. Il se présente de préférence sous la forme d'un film continu disposé en surface du coeur formé en tout ou partie de l'actif. Il possède avantageusement une résistance mécanique suffisante pour être compatible avec une exposition à une force de compression significative par exemple d'au moins 5 kN, notamment d'au moins 7 kN et de préférence supérieure à 10kN.

Cet enrobage est en outre avantageusement homogène en terme de composition, en surface du coeur formant les microparticules.

Ainsi, selon une variante de réalisation préférée, l'enrobage disposé en surface des microparticules est obtenu par pulvérisation en lit d'air fluidisé d'une solution ou dispersion contenant au moins lesdits polymères A et B sur des particules de principe(s) actif(s).

De manière préférentielle, les polymères A et B et si présent(s) le (ou les) plastifiant(s) sont pulvérisés à l'état de soluté c'est-à-dire sous une forme solubilisée dans un milieu solvant. Ce milieu solvant contient généralement des solvants organiques mélangés ou non avec de l'eau. L'enrobage ainsi formé s'avère homogène en terme de composition par opposition à un enrobage formé à partir d'une dispersion de ces mêmes polymères dans un liquide majoritairement aqueux.

Selon une variante de réalisation préférée, la solution pulvérisée contient moins de 40 % en poids d'eau, en particulier moins de 30 % en poids d'eau et plus particulièrement moins de 25 % en poids d'eau, voire une teneur en eau inférieur ou égale à 10 % en poids par rapport au poids total de solvants.

Cette aptitude de l'enrobage à conserver son intégrité physique et ses propriétés de libération modifiée s'observent avantageusement pour des taux d'enrobage variant de 3 à 85 %, notamment de 5 à 60 %, en particulier de 10 à 50 %, voire de 10 à 40 %, et plus particulièrement de 20 à 40 % en poids d'enrobage par rapport au poids total de la microparticule.

Les microparticules considérées selon l'invention possèdent un diamètre moyen inférieur ou égal à 2000 µm, en particulier inférieur ou égal à 1000 µm, notamment inférieur à 800 µm, en particulier inférieur à 600 µm, voire inférieure à 500 µm. Le diamètre moyen est déterminé par diffraction laser ou analyse tamis selon l'échelle de taille à caractériser.

D'une manière générale, l'usage de la méthode par diffraction laser, notamment telle qu'exposée dans Pharmacopée 6ème Edition, chapitre 2.9.31., pour caractériser une taille, en diamètre moyen en volume, est privilégié jusqu'à une échelle de taille de 700 µm. En ce qui concerne la caractérisation selon la méthode tamis, le choix des tamis adéquat relève clairement des compétences de l'homme de l'art qui peut se référer à la Pharmacopée Européenne, 6ème édition, chapitre 2.9.38., proposant un mode d'estimation de la distribution granulométrique par tamisage analytique.

### Actifs

Les formes solides selon l'invention sont compatibles avec une large gamme de principes actifs. Pour des raisons évidentes, leur profil de libération contrôlée en terme de pH et retardée les rend tout particulièrement avantageuses pour des actifs vis-à-vis desquels il est recherché de tels profils de libération et donc plus particulièrement, les actifs dont on cherche à garantir une libération significative au niveau de l'intestin grêle. Tel est le cas de l'essentiel des actifs pharmaceutiques.

Ainsi, l'actif contenu dans les microparticules enrobées selon l'invention peut être, par exemple, avantageusement choisi parmi au moins l'une des familles de substances actives suivantes : les anesthésiques, les analgésiques, les antiasthmatiques, les agents de traitement des allergies, les anticancéreux, les anti-inflammatoires, les anticoagulants et antithrombotiques, les anti-convulsivants, les antiépileptiques, les antidiabétiques, les antiémétiques, les antiglaucomes, les antihistaminiques, les anti-infectieux, notamment les antibiotiques, les antifongiques, les antiviraux, les antiparkinsoniens, les anti-cholinergiques, les antitussifs, les inhibiteurs de l'anhydrase carbonique, les agents cardiovasculaires, notamment les hypolipémiants, anti-arythmiques, vasodilatateurs, antiangineux, anti-hypertenseurs, vasoprotecteurs et inhibiteurs de cholinestérase, les agents de traitement des désordres du système nerveux central, les stimulants du système nerveux central, les contraceptifs, les promoteurs de fécondité, les agonistes des récepteurs de la dopamine, les agents de traitement de l'endométriose, les agents de traitements des troubles gastro-intestinaux, les immunomodulateurs et les immunosuppresseurs, les agents de traitement des troubles de la mémoire, les antimigraineux, les myorelaxants, les analogues de nucléosides, les agents de traitement de l'ostéoporose, les parasympathomimétiques, les prostaglandines, les agents psychothérapeutiques tels que sédatifs, hypnotiques, tranquillisants, neuroleptiques, anxiolytiques, psychostimulants et antidépresseurs, les agents de traitement dermatologiques, les stéroïdes et les hormones, les amphétamines, les anorexigènes, les anti-douleurs non analgésiques, les antiépileptiques, les barbituriques, les benzodiazépines, les hypnotiques, les laxatifs, les psychotropes.

Certaines de ces familles d'actifs sont notamment plus particulièrement illustrées par les actifs mis en oeuvre dans les exemples.

Pour des raisons évidentes, les particules selon l'invention peuvent être mise en oeuvre à des fins de conditionnement d'autres actifs que ceux identifiés ci-dessus.

La forme solide ou composition solide selon l'invention se présente sous la forme d'un comprimé, ce comprimé contenant des microparticules telles que définies ci-dessus.

Selon un mode de réalisation particulier, une forme solide selon l'invention présente un taux de charge en microparticules allant de 5 % à 60 % en poids par rapport à son poids total, en particulier de 10 % à 50 % en poids, et plus particulièrement de 20 à 40 % en poids.

Avantageusement, la forme solide contenant les microparticules à libération modifiée de l'actif comprend également des excipients physiologiquement acceptables, classiques, et utiles par exemple pour formuler les microparticules au sein d'une matrice et notamment sous forme de comprimé.

Ces excipients peuvent être notamment :
- des diluants
- des agents de compression, comme la cellulose microcristalline ou le mannitol,
- des colorants,
- des désintégrants,
- des agents d'écoulement comme le talc, la silice colloïdale,
- des lubrifiants comme par exemple le béhénate de glycérol, les stéarates,
- des arômes,
- des conservateurs,
- et leurs mélanges.

Le choix de ces excipients relève clairement des compétences de l'homme de l'art.

Selon un mode de réalisation particulier de l'invention, les agents de compression et/ou ou diluants sont notamment choisis parmi :
∘ la cellulose microcristalline, comme par exemple les grades d'Avicel® de FMC, les grades de Celphere® d'Asahi Kasei, ou la cellulose poudre,
∘ les sels de calcium, tels que les carbonate, phosphate et sulfate de calcium,
∘ les sucres, comme par exemple le lactose, le sucrose ou les sphères de sucre,
∘ le mannitol, comme par exemple les grades de Pearlitol® de Roquette, le xylitol, et l'erythritol.

Une forme solide selon l'invention peut notamment comprendre un ou plusieurs agent(s) de compression et/ou diluant(s) dans une teneur allant de 10 % à 80 % en poids, en particulier de 30 % à 75 % en poids, et plus particulièrement de 35 % à 65 % en poids par rapport au poids total de la forme solide.

Selon un autre mode de réalisation particulier de l'invention, les lubrifiants et/ou agents d'écoulement sont notamment choisis parmi :
∘ les stéarates, comme par exemple le stéarate de magnésium,
∘ l'acide stéarique,
∘ le béhénate de glycérol,
∘ la silice colloïdale et
∘ le talc.

Une forme solide selon l'invention peut comprendre un ou plusieurs lubrifiant(s) et/ou agent(s) d'écoulement dans une teneur allant de 0,1 % à 5 % en poids, en particulier de 0,5 % à 2 % en poids par rapport au poids total de la forme solide.

Selon un autre mode de réalisation particulier de l'invention, des liants sont notamment choisis parmi :
∘ les polymères dérivés de la cellulose, comme l'hypromellose, la méthylcellulose, l'hydroxypropyl cellulose, l'hydroyethylcellulose, l'éthylcellulose,
∘ la povidone, et
∘ le polyoxyde d'éthylène.

La teneur en liant(s) dans une forme solide selon l'invention peut aller de 0 % à 40 % en poids, en particulier de 0 % à 30 % en poids, et plus particulièrement de 5 à 20 % en poids par rapport au poids total de la forme solide.

Selon un mode de réalisation particulier, une forme solide selon l'invention comprend, outre les microparticules définies ci-dessous, au moins un agent de compression et/ou diluant, en particulier choisi parmi la cellulose microcristalline, le mannitol et leurs mélanges, et au moins un lubrifiant et/ou agent d'écoulement, en particulier du stéarate de magnésium, et éventuellement au moins un liant, notamment choisi parmi l'hypromellose et la méthylcellulose.

En particulier, ces différents excipients sont mis en oeuvre dans des teneurs telles que définies précédemment.

D'autres excipients physiologiquement acceptables peuvent être ajoutés, notamment choisis parmi des désintégrants, des colorants, des arômes et/ou des conservateurs.

Selon un mode de réalisation particulier, une forme solide selon l'invention comprend moins de 1 % en poids de désintégrant(s) par rapport à son poids total, et plus particulièrement, est dépourvue de désintégrant.

Selon encore un autre mode de réalisation particulier, une forme solide selon l'invention est dépourvue de composé cireux insoluble dans l'eau, et en particulier est dépourvue de cires.

La forme solide finale, sous forme de comprimé, peut être enrobée selon les techniques et formules connues de l'homme de l'art pour améliorer sa présentation (couleur, aspect, masquage de goût, etc.).

Les nouvelles formes pharmaceutiques selon l'invention sont originales dans leur aptitude à manifester un profil de libération contrôlée et sont administrables per os, notamment à dose journalière unique, double ou multiple.

Bien entendu, une forme solide selon l'invention peut réunir différents types de microparticules, se distinguant les unes des autres par exemple au regard de la nature de l'actif conditionné, et/ou de la composition de l'enrobage et/ou de l'épaisseur de l'enrobage.

Selon un premier mode de réalisation, au moins une partie des microparticules à libération modifiée de l'actif comporte chacune une microparticule de l'actif, enrobée par au moins un enrobage permettant la libération modifiée de l'actif.

De préférence, la microparticule de l'actif est un granulé comprenant le (les) actif(s) et un ou plusieurs excipients physiologiquement acceptables.

Selon un deuxième mode de réalisation, au moins une partie des microparticules à libération modifiée de l'actif comporte chacune une particule support, au moins une couche active comprenant le (ou les) actif(s) et enrobant la particule support, et au moins un enrobage permettant la libération modifiée de l'actif.

Comme précisé précédemment, il peut être également intéressant de mélanger dans une même forme solide, au moins deux types de microparticules à cinétiques de libération de l'actif différentes, par exemple à libération immédiate et à libération modifiée. Il peut aussi être intéressant de mélanger deux (ou plusieurs) types de microparticules, contenant chacun un actif différent, libéré selon un profil de libération qui lui est spécifique.

La présente invention a également pour objet un procédé de préparation d'une forme solide de type comprimé pour l'administration orale d'au moins un actif, conforme à l'invention comprenant au moins les étapes consistant à :
a) disposer de microparticules formées en tout ou partie d'au moins un actif,
b) pulvériser en lit d'air fluidisé sur les microparticules de l'étape a) une solution ou dispersion contenant au moins un polymère A insoluble dans les fluides gastro-intestinaux en mélange avec au moins un polymère B possédant une valeur de pH de solubilisation comprise dans la plage de pH de 5 à 7, dans un rapport pondéral polymère(s) B/polymère(s) A au moins égal à 0,25,
c) mélanger les microparticules d'actifs, obtenues à l'issue de l'étape b), avec un ou plusieurs excipients physiologiquement acceptables et aptes à former une matrice,
d) agglomérer le mélange formé en étape c) par compression.

Selon une variante de réalisation, les microparticules d'actifs enrobés obtenus à l'issue de l'étape c) peuvent être mélangées avec d'autres microparticules ayant des compositions d'enrobage différentes et/ou des tailles différentes et/ou avec des particules d'actif pur préalablement à leur transformation selon l'étape d).

Les particules d'actifs peuvent au préalable être obtenues selon plusieurs techniques telles que par exemple :
- extrusion/sphéronisation de l'actif avec éventuellement un ou plusieurs excipient(s) physiologiquement acceptables, et/ou ;
- granulation humide de l'actif avec éventuellement un ou plusieurs ou excipient(s) physiologiquement acceptables, et/ou ;
- compactage de l'actif avec éventuellement un ou plusieurs excipient(s) physiologiquement acceptables, et/ou ;
- pulvérisation de l'actif, avec éventuellement un ou plusieurs excipient(s) physiologiquement acceptables, en dispersion ou en solution dans un solvant aqueux ou organique sur une particule support, et/ou ;
- poudre ou cristaux de l'actif éventuellement tamisé(e)(s);
- les microparticules de l'actif peuvent avoir été au préalable enrobées.

Selon une variante de réalisation, la solution en dispersion mise en oeuvre en étape b) est une solution c'est-à-dire un milieu solvant dans lequel les polymères A et B sont à l'état de solutés.

Avantageusement, il s'agit d'un mélange d'eau et de solvant(s) organique(s) dont la teneur en eau est inférieure à 40 % en poids, en particulier inférieure à 30 %, voire inférieure à 25 % en poids, en particulier inférieure ou égale à 10 % en poids par rapport au poids total du mélange de solvants. Le solvant organique peut être choisi parmi les solvants connus de l'homme de l'art. A titre d'exemple, on peut citer les solvants suivants : acétone, isopropanol, éthanol et leurs mélanges.

Les excipients susceptibles d'être associés en étape c) aux microparticules d'actifs peuvent être des diluants, liants, désagrégeants, agents d'écoulement, lubrifiants, composés pouvant modifier le comportement de la préparation dans le tube digestif, colorants et/ou arôme.

Il s'agit là de méthodologies générales intéressantes, qui permettent de produire les formes solides de l'invention d'une manière simple et économique.

Les formes solides selon la présente invention sont avantageusement obtenues par compression. Cette compression peut être réalisée selon toute méthode conventionnelle et sa mise en oeuvre relève clairement des compétences de l'homme de l'art.

Généralement, l'ensemble des ingrédients destinés à former la matrice dans laquelle sont dispersées les microparticules est mélangé à l'état pulvérulent. Ces ingrédients peuvent inclure en outre une ou plusieurs charges, un ou plusieurs lubrifiants, également à l'état de poudre.

Une fois que l'ensemble de ces ingrédients et les particules ont été mélangés, par des méthodes conventionnelles, le mélange résultant est comprimé pour former la forme solide attendue et notamment un comprimé.

La méthode pour préparer de tels comprimés est également bien connue de l'homme de l'art et ne sera donc pas décrite plus en détail ci-après.

Ces comprimés, comme évoqués précédemment, peuvent le cas échéant être soumis à des traitements complémentaires visant par exemple à former à leur surface un pelliculage ou enrobage particulier destiné à leur procurer des propriétés ou qualités complémentaires.

Les exemples et figures qui suivent sont présentés à titre illustratif et non limitatif du domaine de l'invention.

### FIGURES

Figure 1 : Profils comparatifs de libération *in vitro* obtenus dans un milieu 0,1N HCl pour des comprimés, préparés selon l'exemple 2 et des microparticules préparées selon l'exemple 1.
Figure 2 : Profils comparatifs de libération *in vitro* obtenus dans un milieu 0.05M de phosphate de potassium à un pH de 6.8 pour des comprimés, préparés selon l'exemple 2 et des microparticules préparés selon l'exemple 1.
Figure 3 : Profils de libération *in vitro* de microparticules de metformine préparées selon l'exemple 3, obtenus pendant 2 heures dans le milieu 0,1N HCl puis dans le milieu 0.05M de phosphate de potassium à un pH de 6.8
Figure 4 : Profils comparatifs de libération *in vitro* obtenus dans un milieu 0,1N HCl pour des comprimés et des microparticules de metformine, non conformes à l'invention, préparés selon l'exemple 4.
Figure 5 : Profils comparatifs de libération *in vitro* obtenus dans un milieu 0,1N HCl pour des comprimés d'aciclovir, préparés selon l'exemple 6 et des microparticules d'acyclovir, préparées selon l'exemple 5.
Figure 6 : Profils comparatifs de libération *in vitro* obtenus dans un milieu 0,05M de phosphate de potassium à un pH de 6,8 pour des comprimés d'acyclovir, préparés selon l'exemple 6 et des microparticules d'acyclovir, préparées selon l'exemple 5.
Figure 7 : Profils comparatifs de libération *in vitro* obtenus dans un milieu 0,1N HCl comprenant 0,2 % en poids de Cremophor RH 40®, pour des comprimés de diclofénac, préparés selon l'exemple 8 et des microparticules de diclofénac, préparées selon l'exemple 7.
Figure 8 : Profils comparatifs de libération *in vitro* obtenus dans un milieu 0,05M de phosphate de potassium à un pH de 6,8 pour des comprimés de diclofénac, préparés selon l'exemple 8 et des microparticules de diclofénac, préparées selon l'exemple 7.
Figure 9 : Profils de libération *in vitro* obtenus dans un milieu 0,05 M de phosphate à un pH de 6,8 et dans un milieu 0,1 N HCl pour des comprimés préparés selon l'exemple 9.
Figure 10 : Profils comparatifs de libération *in vitro,* obtenus pour des comprimés et des microparticules de metformine, tous deux préparés selon l'exemple 10, lors d'une exposition séquencée à des conditions acides (milieu 0,1N HCl) pendant 2 heures, puis à un pH neutre (medium pH 6,8).

Dans l'ensemble des figures, le symbole ◆ représente le comprimé considéré et le symbole X les microparticules correspondantes et le %D figure le pourcentage dissous.

### Exemple 1

### Préparation et formulation de microparticules de metformine

### Etape 1 : préparation des granulés (étape d'enduction)

1800 g de metformine sont introduits sous agitation dans un réacteur qui contient 2486 g d'eau. La solution est chauffée à 70 °C. Une fois les cristaux de metformine dissous, la solution est pulvérisée sur 200 g de sphères de cellulose (d'Asahi Kasei) dans un lit d'air fluidisé GPCG1.1 dans une configuration bottom spray (pulvérisation de la solution d'enrobage par une buse située dans la partie inférieure du lit de particules).

Après pulvérisation, le produit obtenu est tamisé sur des tamis de 200 µm et 800 µm. 1888 g de granulés allant de 200 µm à 800 µm (ce qui correspond à la fraction de produit ayant traversé les mailles du tamis de 800 µm et retenue sur le tamis de 200 µm) sont alors récupérés.

### Etape 2 : phase d'enrobage

490 g de granulés obtenus en étape 1 sont enrobés à température ambiante, dans un lit d'air fluidisé GPCG 1.1 avec 105 g d'un copolymère d'acide méthacrylique et d'éthyle acrylate 1 :1 (Eudragit L100-55 d'Evonik), 84 g d'acétate butyrate de cellulose (d'Eastman) et 21 g de triéthylcitrate (de Morflex) dissouts dans un mélange acétone/eau (90/10 m/m). Après pulvérisation, les microparticules enrobées sont récupérées. Leur diamètre moyen en volume, déterminé par diffraction laser à l'aide d'un appareil Mastersizer 2000 de Malvern Instrument équipé du module voie sèche Scirocco 2000, selon le mode de calcul « Analyse standard réglée avec une sensibilité normale » (Model : General Purpose - normal sensitivity), est de 631 µm.

### Exemple 2

### Préparation de comprimés contenant les microparticules de l'exemple 1

4,0 g des microparticules à libération différée et contrôlée préparées dans l'exemple 1 sont mélangés à 4,0 g d'hypromellose (Methocel E5 de Dow), 4,0 g de cellulose microcristalline (Avicel PH101 de FMC) et 0,2 g de stéarate de magnésium. Ce mélange est utilisé pour fabriquer des comprimés de 800 mg à l'aide d'une presse hydraulique Perkin-Elmer.

### Tests de dissolution in vitro

La cinétique de libération *in vitro* des comprimés est suivie à 37 ± 0,5 °C par spectrométrie UV, d'une part, dans 900 ml d'un milieu 0,1 N HCl et, d'autre part, dans 900 ml d'un milieu phosphate de potassium 0,05 M à pH 6,8. Les tests de dissolution sont effectués dans un appareil à palettes USP type II. La vitesse de rotation des palettes est de 75 rpm.

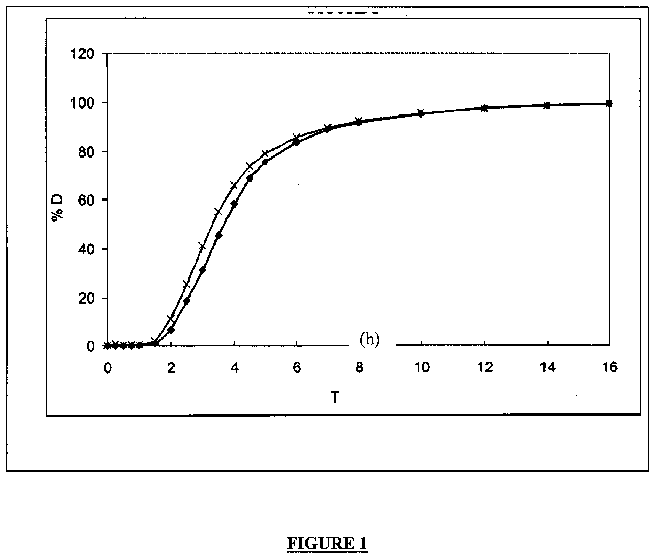

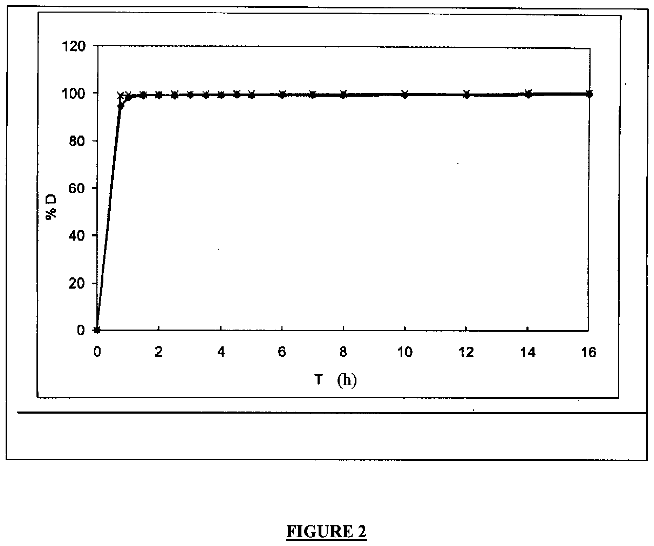

Les résultats sont illustrés respectivement en figures 1 et 2. Chacune des figures 1 et 2 rend également compte à titre comparatif du profil de libération des microparticules sous une forme libre, c'est-à-dire conformes à celles obtenues en exemple 1.

On note que les profils de libération des comprimés et des microparticules sous forme libre sont similaires pour chaque milieu de dissolution testé.

### Exemple 3

### Enrobage de cristaux de metformine

### Phase d'enrobage

420 g de cristaux de metformine, tamisés entre 300 µm et 800 µm, sont enrobés à température ambiante, dans un lit d'air fluidisé GPCG1.1, avec 165 g d'un copolymère d'acide méthacrylique et d'éthyle acrylate 1 :1 (Eudragit L100-55 d'Evonik), 132 g d'acétate butyrate de cellulose (d'Eastman) et 33 g de triéthylcitrate (de Morflex) dissous dans un mélange acétone/eau (90/10 m/m). A l'issue de la pulvérisation, les microparticules attendues sont récupérées. Leur diamètre moyen en volume, déterminé par diffraction laser à l'aide d'un appareil Mastersizer 2000 de Malvern Instrument équipé du module voie sèche Scirocco 2000, selon le mode de calcul « Analyse standard réglée avec une sensibilité normale » (Model : Général Purpose - normal sensitivity), est de 495 µm.

### Profils de dissolution dans des conditions d'expositions séquentielles

La cinétique *in vitro* des microparticules préparées ci-dessus est suivie à 37 °C ± 0,5 °C par spectrométrie UV pendant 2 heures dans un milieu 0,1 N HCl puis, après ajustement du pH, dans un milieu phosphate de potassium 0,05 M à pH 6,8. Le test de dissolution est effectué dans un appareil à palettes USP type II, dans 900 ml de milieu. La vitesse de rotation des palettes est de 75 rpm.

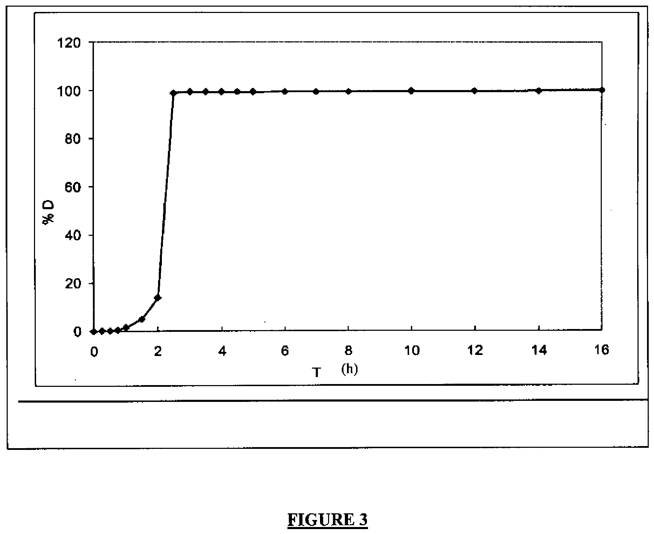

Le profil de dissolution est présenté en Figure 3.

### Exemple 4

### Préparation et formulation de microparticules non conformes à l'invention dans un comprimé

### Phase 1 : préparation des granulés (étape d'enduction)

1746 g de metformine et 54 g de povidone (Plasdone K29/32 de ISP) sont introduits sous agitation dans un réacteur comprenant 2486 g d'eau. La solution est chauffée à 74 °C. Lorsque les cristaux de metformine et la povidone sont dissous, la solution est pulvérisée sur 450 g de sphères de cellulose (d'Asahi Kasei) dans un lit d'air fluidisé GPCG1.1 dans une configuration bottom spray. 2224 g de granulés de metformine sont obtenus.

### Phase 2 : Phase d'enrobage

455 g de granulés, comme préparés ci-dessus, sont enrobés dans un lit d'air fluidisé GPCG 1.1 avec 117 g d'un copolymère d'acide méthacrylique et d'éthyle acrylate 1 :1 (Eudragit L100-55 d'Evonik) et 78 g d'huile de graine de coton hydrogénée (Lubritab® de JRS Pharma), dissouts dans 1305 g d'isopropanol à 78 °C. Après pulvérisation, le produit est porté à 55 °C pendant 2 heures. 638 g de microparticules sont obtenus.

### Préparation des comprimés

4,0 g de microparticules, comme préparées ci-dessus, sont mélangés avec 3,0 g d'hypromellose (Methocel E5 de Dow), 3,0 g de cellulose microcristalline (Avicel PH101 de FMC), 2,0 g de mannitol (Pearlitol SD 200 de Roquette) et 0,2 g de stéarate de magnésium. Ce mélange est utilisé pour fabriquer des comprimés de 800 mg à l'aide d'une presse hydraulique Perkin-Elmer.

### Tests de dissolution in vitro

La cinétique de libération *in vitro* des microparticules de metformine et des comprimés préparés comme décrit précédemment est suivie à 37 ± 0,5 °C par spectrométrie UV dans 900 ml de milieu 0,1 N HCl. Les tests de dissolution sont effectués dans un appareil à palettes USP type II. La vitesse de rotation des palettes est de 75 rpm.

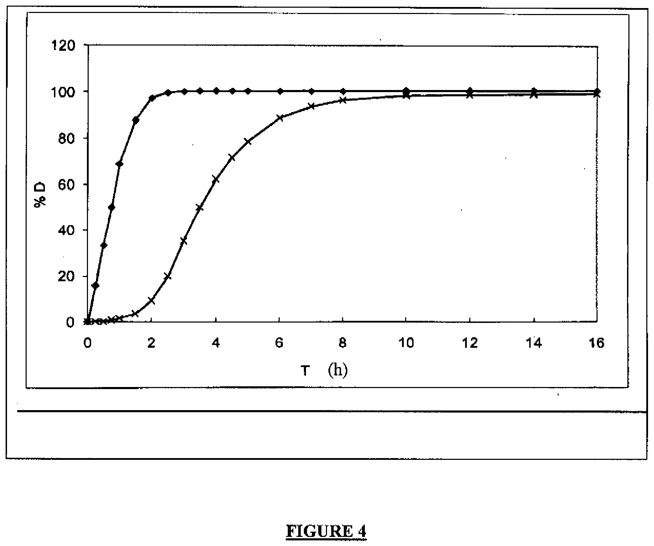

Les profils de dissolution sont illustrés en figure 4. On note que les profils de libération des comprimés et des microparticules sous forme libre sont différents. Le profil de libération du comprimé n'est pas conforme à celui des microparticules. Il est plus rapide, traduisant un défaut de contrôle.

### Exemple 5 (Hors invention)

### Fabrication de microparticules d'aciclovir

### Phase 1 : préparation des granulés (étape d'enduction)

810 g d'aciclovir et 90 g de povidone (Plasdone K29/32 de ISP) sont introduits sous agitation dans 2100 g d'eau. Lorsque les cristaux d'aciclovir et la povidone sont dissous, la solution est pulvérisée sur 600 g de sphères de cellulose (d'Asahi Kasei) dans un lit d'air fluidisé GPCG1.1 dans une configuration bottom spray. Après enduction, le produit est tamisé sur des tamis d'ouverture de maille de 200 µm et 800 µm. Des granulés d'aciclovir allant de 200 µm à 800 µm, correspondant à la fraction de produit passée à travers les mailles du tamis de 800µm et récupérée sur le tamis de 200 µm, sont obtenus.

### Phase 2 : Phase d'enrobage

350 g de granulés, comme préparés ci-dessus, sont enrobés à température ambiante dans un lit d'air fluidisé GPCG 1.1 avec 45 g d'un copolymère d'acide méthacrylique et d'éthyle acrylate 1:1 (Eudragit L100-55 d'Evonik), 90 g d'un copolymère d'ammonio (méth)acrylate type A (Eudragit RL100 d'Evonik) et 15 g de dibutylphtalate (de Merck), dissouts dans un mélange acétone/eau (90/10 m/m). Après pulvérisation, des microparticules sont obtenues. Le diamètre moyen en volume des microparticules d'aciclovir enrobées, déterminé par diffraction laser sur un appareil Mastersizer 2000 de Malvern Instrument équipé du module voie sèche Scirocco 2000, selon le mode de calcul « Analyse standard réglée avec une sensibilité normale » (Model : General Purpose - normal sensitivity), est de 412 µm.

### Exemple 6 (Hors invention)

### Dissolution à 0,1N HCl et à un pH de 6.8 de comprimés comprenant des microparticules d'aciclovir

2,0 g de microparticules à libération différée et contrôlée, comme celles préparées dans l'exemple 5, sont mélangés à 1,0 g d'hypromellose (Methocel E5 de Dow), 2,0 g de cellulose microcristalline (Avicel PH101 de FMC) et 1,0 g de mannitol (Pearlitol SD200 de Roquette) et 0,1 g de stéarate de magnésium. Ce mélange est utilisé pour la fabrication de comprimés pesant 800 mg.

La cinétique de libération *in vitro* des comprimés est suivie à 37 ± 0,5 °C par spectrométrie UV, d'une part dans 900 ml d'un milieu 0,1 N HCl et, d'autre part dans 900 ml d'un milieu phosphate de potassium 0,05M à un pH de 6.8. Les tests de dissolution sont effectués dans un appareil à palettes USP type II. La vitesse de rotation des palettes est de 75 rpm.

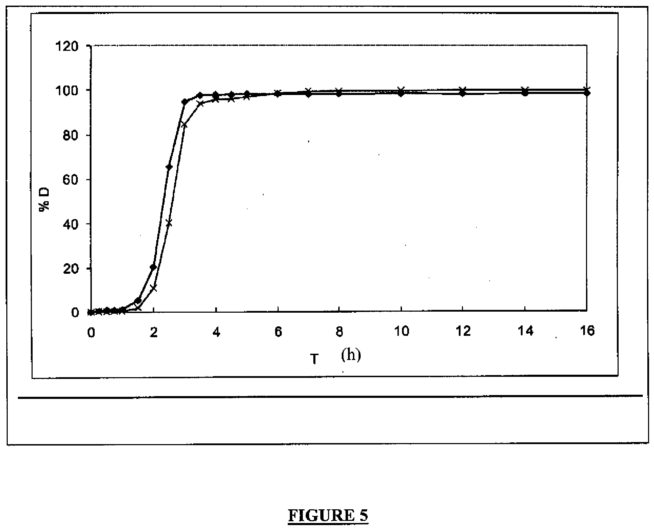

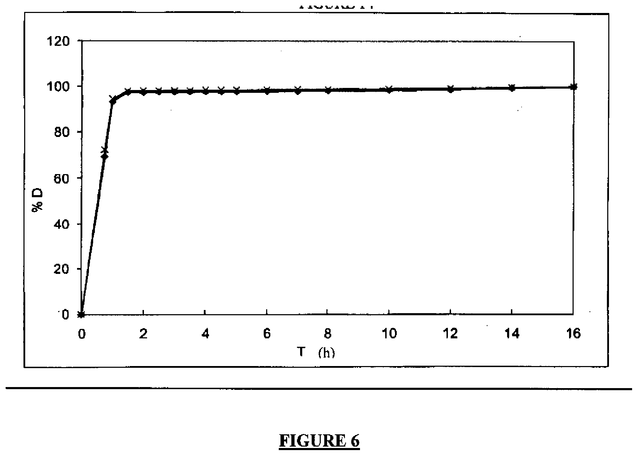

Les profils de dissolution des comprimés sont comparés en figures 5 et 6 aux profils de dissolution des microparticules d'aciclovir préparées en exemple 5.

Les profils de libération des comprimés et des microparticules sous forme libre sont identiques pour chaque milieu de dissolution testé.

### Exemple 7

### Fabrication de microparticules de diclofénac

### Phase 1 : préparation des granulés (étape d'enduction)

100 g de diclofénac de sodium et 400 g de povidone (Plasdone K29/32 de ISP) sont introduits sous agitation dans 1674 g d'eau. La solution est chauffée à 70 °C. Après dissolution complète des ingrédients, la solution est pulvérisée sur 600 g de sphères de cellulose (d'Asahi Kasei) dans un lit d'air fluidisé GPCG1.1 dans une configuration bottom spray. Les granulés obtenus sont tamisés sur des tamis de 200 µm à 500 µm. Des granulés de diclofénac allant de 200 µm à 500 µm sont obtenus.

### Phase 2 : Phase d'enrobage

420 g de granulés, comme préparés ci-dessus, sont enrobés à température ambiante dans un lit d'air fluidisé GPCG1.1, avec une solution comprenant 108 g d'un copolymère d'acide méthacrylique et d'éthyle acrylate 1 :1 (Eudragit L100-55 d'Evonik), 54 g d'un copolymère d'ammonio (méth)acrylate type B (Eudragit RS100 d'Evonik), 18 g de triéthylcitrate (de Morflex), dissous dans un mélange acétone/eau (95/5 m/m). Après pulvérisation, le produit est tamisé sur 630 µm. Les microparticules ainsi obtenues ont un diamètre moyen en volume, déterminé par diffraction laser à l'aide d'un appareil Mastersizer 2000 de Malvern Instrument équipé du module voie sèche Scirocco 2000, selon le mode de calcul « Analyse standard réglée avec une sensibilité normale » (Model : General Purpose - normal sensitivity), de 411 µm.

### Exemple 8

### Dissolution de comprimés comprenant des microparticules de diclofénac

112,5 g de microparticules à libération différée et contrôlée, comme celles préparées dans l'exemple 7, sont mélangés à 157,8 g de cellulose microcristalline (Avicel PH101 de FMC), 28,2 g de mannitol (Pearlitol SD200 de Roquette) et 1,5 g de stéarate de magnésium. Ce mélange est utilisé pour la fabrication de comprimés ronds de diamètre 12 mm de 700 mg, grâce à une presse XP1 de Korsch. La force de compression appliquée sur le mélange est de 15 kN. Les comprimés ainsi fabriqués ont une dureté d'environ 98 N.

La cinétique de libération *in vitro* des comprimés ci-dessus est suivie à 37 ± 0,5 °C par spectrométrie UV, d'une part, dans 900 ml d'un milieu 0,1 N HCl contenant 0,2 % massique de Cremophor RH 40, et, d'autre part, dans 900 ml d'un milieu phosphate de potassium 0,05M à pH 6.8. Les tests de dissolution sont effectués dans un appareil à palettes USP type II. La vitesse de rotation des palettes est de 75 rpm.

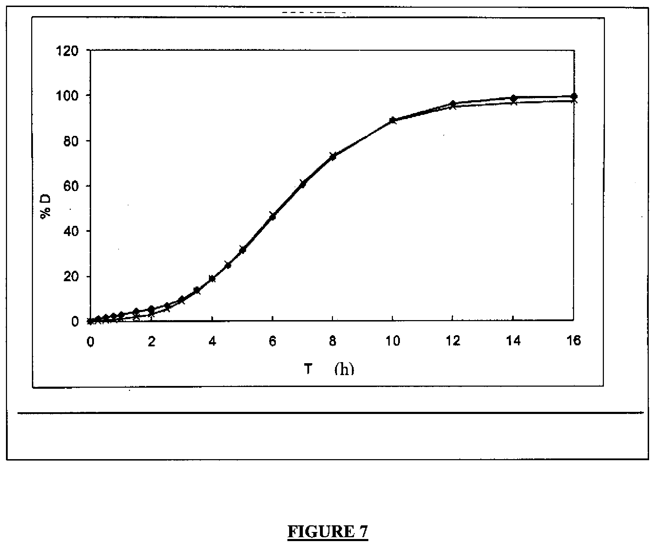

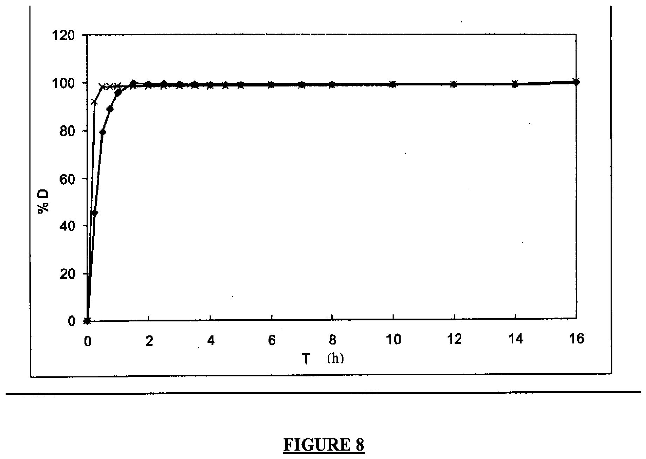

Les profils de dissolution des comprimés sont comparés en figures 7 et 8 aux profils de dissolution des microparticules de diclofénac préparées en exemple 7.

Les profils de libération des comprimés et des microparticules sous forme libre sont identiques pour chaque milieu de dissolution testé.

### Exemple 9

### Préparation de comprimés contenant les granulés non enrobés et les microparticules de l'exemple 1

1,0 g de granulés non enrobés préparés dans l'exemple 1 et 3,0 g des microparticules à libération différée et contrôlée préparées dans l'exemple 1, sont mélangés à 5,0 g de cellulose microcristalline (Avicel PH101 de FMC), 0,9 g de mannitol (Pearlitol SD 100 de Roquette) et 0,1 g de stéarate de magnésium. Ce mélange est utilisé pour fabriquer des comprimés de 800 mg à l'aide d'une presse hydraulique Perkin-Elmer.

### Tests de dissolution in vitro

La cinétique de libération *in vitro* des comprimés est suivie à 37 ± 0,5 °C par spectrométrie UV, d'une part, dans 900 ml d'un milieu 0,1 N HCl et, d'autre part, dans 900 ml d'un milieu phosphate de potassium 0,05M à pH 6,8. Les tests de dissolution sont effectués dans un appareil à palettes USP type II. La vitesse de rotation des palettes est de 75 rpm.

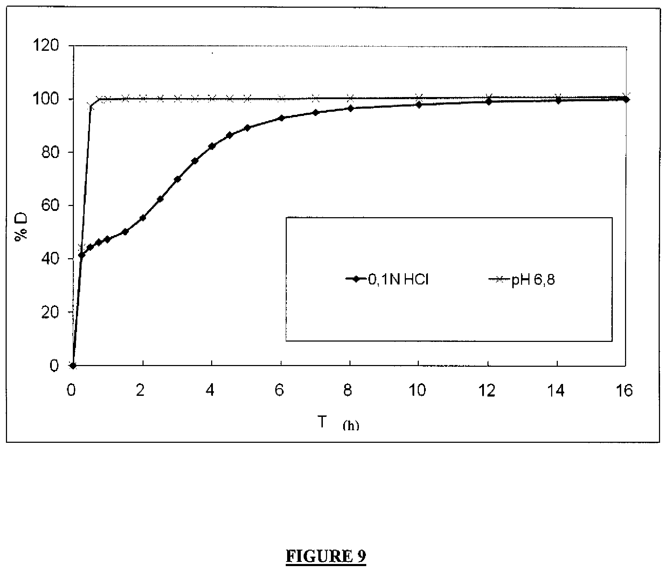

Les résultats sont présentés en figure 9.

Dans le milieu à 0.1N HCl, la fraction de principe actif libérée immédiatement correspond à la fraction de principe actif contenue dans les granulés non enrobés utilisés pour la fabrication des comprimés.

### Exemple 10

### Préparation de microparticules et de comprimés de metformine

### Phase d'enrobage

420 g de granulés obtenus en étape 1 de l'exemple 1 sont enrobés à température ambiante, dans un lit d'air fluidisé GPCG1.1, avec 37 g d'un copolymère d'acide méthacrylique et d'acrylate d'éthyle (Eudragit L100-55 d'Evonik), 29.6 g d'éthyl cellulose (Ethocel 20 premium de Dow) et 7.4 g de triéthylcitrate (de Morflex) dissous dans un mélange acétone/eau (90/10 m/m). Après pulvérisation, les microparticules enrobées sont récupérées. Leur diamètre moyen en volume, déterminé par diffraction laser à l'aide d'un appareil Mastersizer 2000 de Malvern Instrument équipé du module voie sèche Scirocco 2000, est de 640 µm.

### Préparation des comprimés

2,0 g des microparticules à libération différée et contrôlée préparées dans l'étape précédente, sont mélangés à 2,0 g d'hypromellose (Methocel E5 de Colorcon), 3,0 g de cellulose microcristalline (Avicel PH101 de FMC), 3,0 g de mannitol (Pearlitol SD 200 de Roquette) et 0,2 g de stéarate de magnésium. Ce mélange est utilisé pour fabriquer des comprimés de 800 mg à l'aide d'une presse hydraulique Perkin-Elmer.

Remarque : On peut également obtenir de manière similaire des comprimés contenant des microparticules préparées comme décrit ci-dessus, mais en substituant les 37 g d'Eudragit L100-55 par un mélange de 14,8 g d'Eudragit L100-55 et 22,2 g d'Eudragit S100.

### Profils de dissolution dans des conditions d'expositions séquentielles

Les profils de dissolution *in vitro* des comprimés et des microparticules, préparés ci-dessus, sont suivis à 37, ± 0,5 °C par spectrométrie UV dans 900 ml de HCl à 0,1 N pendant 2 heures puis, après ajustement du pH et de la salinité du milieu, à pH 6.8 et à 0.05M de phosphate de potassium. Le test de dissolution est effectué dans un appareil à palettes USP type II. La vitesse de rotation des palettes est de 75 rpm.

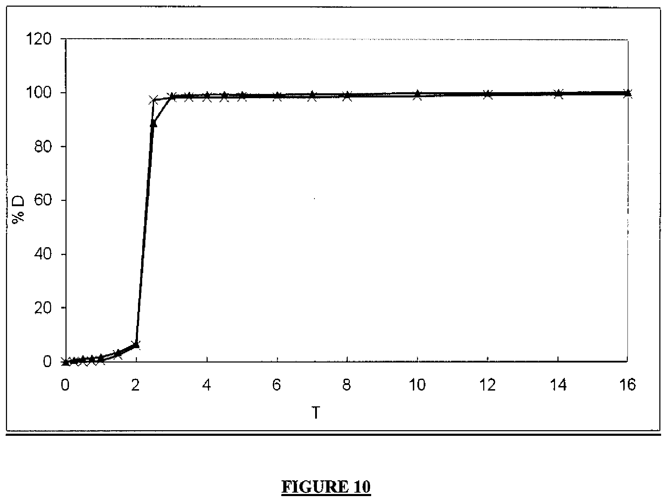

Les profils de dissolution obtenus pour les microparticules et les comprimés sont comparés en figure 10.

## Revendications

1. Comprimé, destiné à l'administration par voie orale d'au moins un actif et apte à garantir un double mécanisme de libération dudit actif, le premier étant conditionné par le temps et le second étant conditionné par le pH,
dans lequel ledit actif y est présent sous la forme d'un système microparticulaire dont les microparticules possèdent un coeur formé en tout ou partie dudit actif et enrobé d'au moins une couche conditionnant ledit profil de libération dudit actif et formée d'un matériau composé d'au moins :
- 30 à 50% en poids par rapport au poids total dudit enrobage d'au moins un polymère A insoluble dans les fluides gastro-intestinaux, choisi parmi l'éthylcellulose, l'acétate butyrate de cellulose, l'acétate de cellulose, les copolymères d'ammonio (méth)acrylate de type « A » ou de type « B », les esters d'acides poly(méth)acryliques, et leurs mélanges ;
- 30 à 60% en poids par rapport au poids total dudit enrobage d'au moins un polymère B possédant une valeur de pH de solubilisation comprise dans la plage de pH de 5 à 7, choisi parmi :
∘ le(s) copolymère(s) d'acide méthacrylique et de méthacrylate de méthyle,
∘ le(s) copolymère(s) d'acide méthacrylique et d'acrylate d'éthyle,
∘ les dérivés cellulosiques tels que l'acétate phtalate de cellulose, l'acétate succinate de cellulose, l'acétate trimellilate de cellulose, le phtalate d'hydroxypropylméthylcellulose, l'acétate succinate d'hydroxypropyl méthylcellulose,
∘ la gomme shellac,
∘ le polyvinyle d'acétate phtalate, et
∘ leurs mélanges, et
ladite couche d'enrobage des microparticules étant composée d'une unique couche formée dudit matériau ;
lesdites microparticules étant dispersées au sein du comprimé,
lesdites microparticules étant aptes à libérer ledit actif selon un profil de libération modifiée en trois phases **caractérisé par** :
- au contact d'un milieu dont le pH est à une valeur inférieure à celle du pH de solubilisation du polymère B, un profil de libération retardée et prolongée avec un temps de latence donné et compris entre 0,5 et 12 heures, en particulier entre 0,5 et 8 heures, voire entre 1 et 5 heures et selon un temps de demie libération t_{1/2} compris entre 0,75 et 24 heures, notamment entre 0,75 et 12 heures, voire entre 0,75 et 8 heures, en particulier entre 1 et 5 heures, temps au terme duquel la moitié de l'actif contenu est libérée ;
- au contact d'un milieu dont le pH est à une valeur supérieure à celle du pH de solubilisation du polymère B, une libération de l'actif sans temps de latence et avec un t_{1/2} compris entre 0,1 et 2 heures ;
la cinétique de libération de l'actif pouvant être déterminée par des tests de dissolution *in vitro*, effectués dans un appareil à palettes USP type II avec une vitesse de rotation des palettes de 75 rpm, par spectrométrie UV, d'une part, dans un milieu 0,1 N HCl et, d'autre part, dans un milieu phosphate de potassium 0,05 M à pH 6,8.

2. Comprimé selon la revendication précédente, dans lequel l'enrobage des microparticules contient de 40 % à 60 % en poids de polymère(s) B par rapport au poids total dudit enrobage.

3. Comprimé selon l'une quelconque des revendications précédentes, dans lequel l'enrobage des microparticules est formé d'au moins un mélange d'éthylcellulose, d'acétate butyrate de cellulose ou de copolymère d'ammonio (méth)acrylate de type « A » ou « B » avec au moins un copolymère d'acide méthacrylique et d'acrylate d'éthyle ou un copolymère d'acide méthacrylique et de méthacrylate de méthyle ou un de leurs mélanges.

4. Comprimé selon l'une quelconque des revendications précédentes, dans lequel l'enrobage comprend les polymères A et B dans un rapport pondéral polymère(s) B/polymère(s) A supérieur ou égal à 0,75.

5. Comprimé selon l'une quelconque des revendications précédentes, dans lequel l'enrobage des microparticules comprend en outre au moins un agent plastifiant.

6. Comprimé selon la revendication précédente, dans lequel l'enrobage des microparticules comprend moins de 25 % en poids, en particulier de 1 % à 20 % en poids, et plus préférentiellement de 5 % à 20 % en poids d'agent(s) plastifiant(s) par rapport à son poids total.

7. Comprimé selon l'une quelconque des revendications précédentes, dans lequel l'enrobage disposé en surface des microparticules, est présent à un taux d'enrobage variant de 3 à 85 % en poids, notamment de 5 à 60 % en poids, en particulier de 10 à 50 %, voire de 10 à 40 %, et plus particulièrement de 20 à 40 % en poids d'enrobage, par rapport au poids total de ladite microparticule.

8. Comprimé selon l'une quelconque des revendications précédentes, dans lequel l'enrobage disposé en surface des microparticules est obtenu par pulvérisation en lit d'air fluidisé d'une solution contenant au moins lesdits polymères A et B à l'état de soluté sur des particules d'actif(s).

9. Comprimé selon l'une quelconque des revendications précédentes, dans lequel les microparticules possèdent un diamètre moyen inférieur ou égal à 2000 µm, en particulier inférieur ou égal à 1000 µm, notamment inférieur à 800 µm, en particulier inférieur à 600 µm, voire inférieur à 500 µm.

10. Comprimé selon l'une quelconque des revendications précédentes, dans lequel l'actif est choisi parmi les anesthésiques, les analgésiques, les antiasthmatiques, les agents de traitement des allergies, les anticancéreux, les anti- inflammatoires, les anticoagulants et antithrombotiques, les anti-convulsivants, les antiépileptiques, les antidiabétiques, les antiémétiques, les antiglaucomes, les antihistaminiques, les anti-infectieux, notamment les antibiotiques, les antifongiques, les antiviraux, les antiparkinsoniens, les anti-cholinergiques, les antitussifs, les inhibiteurs de l'anhydrase carbonique, les agents cardiovasculaires, notamment les hypolipémiants, anti-arythmiques, vasodilatateurs, antiangineux, anti-hypertenseurs, vasoprotecteurs et inhibiteurs de cholinestérase, les agents de traitement des désordres du système nerveux central, les stimulants du système nerveux central, les contraceptifs, les promoteurs de fécondité, les agonistes des récepteurs de la dopamine, les agents de traitement de l'endométriose, les agents de traitements des troubles gastro-intestinaux, les immunomodulateurs et les immunosuppresseurs, les agents de traitement des troubles de la mémoire, les antimigraineux, les myorelaxants, les analogues de nucléosides, les agents de traitement de l'ostéoporose, les parasympathomimétiques, les prostaglandines, les agents psychothérapeutiques tels que sédatifs, hypnotiques, tranquillisants, neuroleptiques, anxiolytiques, psychostimulants et antidépresseurs, les agents de traitement dermatologiques, les stéroïdes et les hormones, les amphétamines, les anorexigènes, les anti-douleurs non analgésiques, les antiépileptiques, les barbituriques, les benzodiazépines, les hypnotiques, les laxatifs, les psychotropes.

11. Comprimé selon l'une quelconque des revendications précédentes, comprenant au moins deux types de microparticules se distinguant l'un de l'autre par des profils de libération distincts.

12. Comprimé selon l'une quelconque des revendications précédentes, comprenant au moins deux types de microparticules, dans lequel lesdits types diffèrent entre eux au moins par la nature de l'actif qu'ils contiennent et/ou par la composition de leur enrobage et/ou l'épaisseur de leur enrobage.

13. Procédé de préparation d'un comprimé pour l'administration orale d'au moins un actif selon l'une quelconque des revendications précédentes, comprenant au moins les étapes consistant à :
a) disposer de microparticules formées en tout ou partie d'au moins un actif,
b) pulvériser en lit d'air fluidisé sur les microparticules de l'étape a), une solution ou dispersion contenant au moins un polymère A insoluble dans les fluides gastro-intestinaux en mélange avec au moins un polymère B possédant une valeur de pH de solubilisation comprise dans la plage de pH de 5 à 7,
c) mélanger les microparticules d'actifs enrobées obtenues à l'issue de l'étape b) avec un ou plusieurs excipients physiologiquement acceptables et aptes à former une matrice,
d) agglomérer le mélange formé en étape c) par compression.

14. Procédé selon la revendication précédente, dans lequel les polymères A et B sont tels que définis en revendications 1 à 4.

15. Procédé selon la revendication 13 ou 14 dans lequel les microparticules obtenues à l'issue de l'étape b) sont telles que définies en revendications 5 à 9.

16. Microparticules possédant un coeur formé en tout ou partie d'au moins un actif, ledit coeur étant enrobé d'au moins une couche conditionnant un double mécanisme de libération dudit actif, le premier étant conditionné par le temps et le second étant conditionné par le pH,
ladite couche d'enrobage étant formée d'un matériau composé d'au moins :
- 30 à 50 % par rapport au poids total dudit enrobage d'au moins un polymère A insoluble dans les fluides gastro-intestinaux et choisi parmi l'éthylcellulose, l'acétate butyrate de cellulose, un copolymère d'ammonio (méth)acrylate de type « A » ou de type « B », les esters d'acides poly(méth)acrylique, et leurs mélanges, et
-- 40 à 60 % en poids par rapport au poids total dudit enrobage d'au moins un polymère B possédant une valeur de pH de solubilisation comprise dans la plage de pH variant de 5 à 7 et choisi parmi un copolymère d'acide méthacrylique et de méthacrylate de méthyle, un copolymère d'acide méthacrylique et d'acrylate d'éthyle et leurs mélanges ;
ladite couche d'enrobage des microparticules étant composée d'une unique couche formée dudit matériau,
lesdites microparticules étant aptes à libérer ledit actif selon un profil de libération modifiée en trois phases **caractérisé par** :
- au contact d'un milieu dont le pH est à une valeur inférieure à celle du pH de solubilisation du polymère B, un profil de libération retardée et prolongée avec un temps de latence donné et compris entre 0,5 et 12 heures, en particulier entre 0,5 et 8 heures, voire entre 1 et 5 heures et selon un temps de demie libération t_{1/2} compris entre 0,75 et 24 heures, notamment entre 0,75 et 12 heures, voire entre 0,75 et 8 heures, en particulier entre 1 et 5 heures, temps au terme duquel la moitié de l'actif contenu est libérée ;
- au contact d'un milieu dont le pH est à une valeur supérieure à celle du pH de solubilisation du polymère B, une libération de l'actif sans temps de latence et avec un t_{1/2} compris entre 0,1 et 2 heures ;
la cinétique de libération dudit actif pouvant être déterminée par des tests de dissolution in vitro, effectués dans un appareil à palettes USP type II avec une vitesse de rotation des palettes de 75 rpm, par spectrométrie UV, d'une part, dans un milieu 0,1 N HCl et, d'autre part, dans un milieu phosphate de potassium 0,05 M à pH 6,8.

17. Microparticules selon la revendication précédente, dont l'enrobage est formé d'au moins un couple polymère B/polymère A choisi parmi les couples suivants :
- copolymère d'acide méthacrylique et d'acrylate d'éthyle, 1:1 / éthylcellulose,
- copolymère d'acide méthacrylique et de méthacrylate de méthyle 1:2 / éthylcellulose,
- mélange de copolymère d'acide méthacrylique et d'acrylate d'éthyle 1:1 et de copolymère d'acide méthacrylique et de méthacrylate de méthyle, 1:2 / éthylcellulose,
- copolymère d'acide méthacrylique et d'acrylate d'éthyle 1:1 / acétate butyrate de cellulose,
- copolymère d'acide méthacrylique et de méthacrylate de méthyle 1:2 / acétate butyrate de cellulose,
- mélange de copolymère d'acide méthacrylique et d'acrylate d'éthyle 1:1 et de copolymère d'acide méthacrylique et de méthacrylate de méthyle 1:2 / acétate butyrate de cellulose,
- copolymère d'acide méthacrylique et d'acrylate d'éthyle 1:1 / copolymère d'ammonio (méth)acrylate de type « A » ou type « B »,
- copolymère d'acide méthacrylique et de méthacrylate de méthyle 1:2 / copolymère d'ammonio (méth)acrylate de type « A » ou type « B »,
- mélange de copolymère d'acide méthacrylique et d'acrylate d'éthyle 1:1 et de copolymère d'acide méthacrylique et de méthacrylate de méthyle 1:2 / copolymère d'ammonio (méth)acrylate de type « A » ou type « B ».

## Patentansprüche

1. Tablette, die zur oralen Verabreichung von mindestens einem Wirkstoff bestimmt ist und einen dualen Freisetzungsmechanismus des Wirkstoffs gewährleisten kann, wobei der erste durch die Zeit bedingt ist und der zweite durch den pH-Wert bedingt ist,
wobei der Wirkstoff dort in Form eines Mikropartikelsystems vorliegt, dessen Mikropartikel einen Kern aufweisen, der ganz oder teilweise aus dem genannten Wirkstoff gebildet ist, und mit mindestens einer Schicht beschichtet ist, die das Freisetzungsprofil des Wirkstoffs bedingt und aus einem Material gebildet ist, bestehend aus mindestens:
- 30 bis 50 Gew.-%, bezogen auf das Gesamtgewicht der Beschichtung, mindestens eines Polymers A, das in den gastrointestinalen Flüssigkeiten unlöslich ist, ausgewählt aus Ethylcellulose, Celluloseacetatbutyrat, Celluloseacetat, Ammonium(meth)acrylat-Copolymeren Typ "A" oder Typ "B", Poly(meth)acrylsäureestern und Mischungen davon;
- 30 bis 60 Gew.-%, bezogen auf das Gesamtgewicht der Beschichtung, mindestens eines Polymers B, das einen Solubilisierungs-pH-Wert im pH-Bereich zwischen 5 bis 7 aufweist, ausgewählt aus:
∘ Methacrylsäure/Methylmethacrylat-Copolymer(en),
∘ Methacrylsäure/Ethylacrylat-Copolymer(en),
∘ Cellulosederivaten, wie etwa Celluloseacetatphthalat, Celluloseacetatsuccinat, Celluloseacetattrimellilat, Hydroxypropylmethylcellulosephthalat,
Hydroxypropylmethylcelluloseacetatsuccinat,
∘ Schellackgummi,
∘ Polyvinylacetatphthalat und
∘ Mischungen davon und
wobei die Beschichtungsschicht der Mikropartikel aus einer einzigen Schicht besteht, die durch das Material gebildet wird;
wobei die Mikropartikel in der Tablette dispergiert sind,
wobei die Mikropartikel den Wirkstoff gemäß einem modifizierten Freisetzungsprofil in drei Phasen freisetzen können, **gekennzeichnet durch**:
- bei Kontakt mit einem Medium, dessen pH-Wert einen Wert aufweist, der kleiner ist als der des Solublisierungs-pH-Werts des Polymers B, ein verzögertes und verlängertes Freisetzungsprofil mit einer gegebenen Latenzzeit, die im Bereich zwischen 0,5 und 12 Stunden, insbesondere zwischen 0,5 und 8 Stunden oder sogar zwischen 1 und 5 Stunden und gemäß einer halben Freisetzungszeit t_{1/2} im Bereich zwischen 0,75 und 24 Stunden, insbesondere zwischen 0,75 und 12 Stunden, oder sogar zwischen 0,75 und 8 Stunden, insbesondere zwischen 1 und 5 Stunden liegt, einer Zeit, an deren Ende die Hälfte des enthaltenen Wirkstoff freigesetzt ist;
- bei Kontakt mit einem Medium, dessen pH-Wert ein Wert ist, der größer ist als der des Solubilisierungs-pH-Werts des Polymers B, eine Freisetzung des Wirkstoffs ohne Latenzzeit und mit einem t_{1/2} im Bereich zwischen 0,1 und 2 Stunden;
wobei die Freisetzungskinetik des Wirkstoffs mit In-vitro-Auflösungstests bestimmt werden kann, die in einem USB-II-Gerät mit Quirlen mit einer Drehgeschwindigkeit der Quirle von 75 U/min, mittels UV-Spektrometrie einerseits in einem 0,1N-HCl-Medium und andererseits in einem 0,05M-Kaliumphosphat-Medium bei einem pH-Wert von 6,8 durchgeführt werden.

2. Tablette nach dem vorhergehenden Anspruch, wobei die Beschichtung der Mikropartikel 40 Gew.-% bis 60 Gew.-% Polymer(e) B, bezogen auf das Gesamtgewicht der Beschichtung, enthält.

3. Tablette nach einem der vorhergehenden Ansprüche, wobei die Beschichtung der Mikropartikel aus mindestens einer Mischung aus Ethylcellulose, Celluloseacetatbutyrat oder Ammonium(meth)acrylat-Copolymer Typ "A" oder "B" mit mindestens einem Methacrylsäure/Ethylacrylat-Copolymer oder einem Methacrylsäure/Methylmethacrylat-Copolymer oder einer Mischung davon gebildet ist.

4. Tablette nach einem der vorhergehenden Ansprüche, wobei die Beschichtung die Polymere A und B in einem Polymer(e)-B/Polymer(e)-A-Gewichtsverhältnis von größer oder gleich 0,75 umfasst.

5. Tablette nach einem der vorhergehenden Ansprüche, wobei die Beschichtung der Mikropartikel ferner mindestens einen Weichmacher umfasst.

6. Tablette nach dem vorhergehenden Anspruch, wobei die Beschichtung der Mikropartikel weniger als 25 Gew.-%, insbesondere 1 Gew.-% bis 20 Gew.-% und besonders bevorzugt 5 Gew.-% bis 20 Gew.-% Weichmacher, bezogen auf ihr Gesamtgewicht, umfasst.

7. Tablette nach einem der vorhergehenden Ansprüche, wobei die auf der Oberfläche der Mikropartikel angeordnete Beschichtung in einem Beschichtungsanteil vorhanden ist, das zwischen 3 bis 85 Gew.-%, insbesondere 5 bis 60 Gew.-%, insbesondere 10 bis 50 Gew.-%, oder sogar 10 bis 40 Gew.-% und insbesondere 20 bis 40 Gew.-% Beschichtung, bezogen auf das Gesamtgewicht des Mikropartikels, variiert.

8. Tablette nach einem der vorhergehenden Ansprüche, wobei die auf der Oberfläche der Mikropartikel angeordnete Beschichtung erhalten wird, indem eine Lösung, die mindestens die Polymere A und B in gelöstem Zustand enthält, auf die Wirkstoffpartikel in einer Wirbelschicht gesprüht wird.

9. Tablette nach einem der vorhergehenden Ansprüche, wobei die Mikropartikel einen mittleren Durchmesser von kleiner oder gleich 2.000 µm, insbesondere kleiner oder gleich 1.000 µm, insbesondere kleiner 800 µm, speziell kleiner 600 µm oder sogar kleiner 500 µm aufweisen.

10. Tablette nach einem der vorhergehenden Ansprüche, wobei der Wirkstoff ausgewählt ist aus Anästhetika, Analgetika, Antiasthmatika, Allergiebehandlungsmitteln, Antineoplastika, Entzündungshemmern, Antikoagulanzien und Antithrombotika, Antikonvulsiva, Antiepileptika, Antidiabetika, Antiemetika, Antiglaukomwirkstoffen, Antihistaminika, Antiinfektiva, insbesondere Antibiotika, Antimykotika, Virostatika, Antiparkinsonmitteln, Anticholinergika, Antitussiva, Carboanhydrase-Inhibitoren, Herz-Kreislaufmitteln, insbesondere Cholesterinsenkern, Antiarrhythmika, Vasodilatatoren, Antianginosa, Antihypertonika, Vasoprotektiva und Cholinesteraseinhibitoren, Mitteln zur Behandlung von Störungen des Zentralnervensystems, Stimulanzien des Zentralnervensystems, Kontrazeptiva, Fruchtbarkeitsförderern, Dopaminrezeptoragonisten, Mitteln zur Behandlung von Endometriose, Mitteln zur Behandlung von gastrointestinalen Störungen, Immunmodulatoren und Immunsuppressoren, Mitteln zur Behandlung von Gedächtnisstörungen, Antimigränemedikamenten, Myorelaxanzien, Nukleosidanaloga, Mitteln zur Behandlung von Osteoporose, Parasympathomimetika, Prostaglandinen, Psychotherapeutika, wie etwa Sedativa, Hypnotika, Beruhigungsmitteln, Neuroleptika, Anxiolytika, Psychostimulanzien und Antidepressiva, Mitteln zur dermatologischen Behandlung, Steroiden und Hormonen, Amphetaminen, Anorektika, nicht analgetischen Schmerzmitteln, Antiepileptika, Barbituraten, Benzodiazepinen, Hypnotika, Abführmitteln, Psychopharmaka.

11. Tablette nach einem der vorhergehenden Ansprüche, umfassend mindestens zwei Arten von Mikropartikeln, die sich durch verschiedene Freisetzungsprofile voneinander unterscheiden.

12. Tablette nach einem der vorhergehenden Ansprüche, umfassend mindestens zwei Arten von Mikropartikeln, wobei sich die Arten mindestens in der Beschaffenheit des Wirkstoffs, den sie enthalten und/oder in der Zusammensetzung ihrer Beschichtung und/oder der Dicke ihrer Beschichtung unterscheiden.

13. Verfahren zur Herstellung einer Tablette zur oralen Verabreichung mindestens eines Wirkstoffs nach einem der vorhergehenden Ansprüche, umfassend mindestens die Schritte:
a) Bereitstellen von Mikropartikeln, die ganz oder teilweise aus mindestens einem Wirkstoff gebildet sind,
b) Aufsprühen in der Wirbelschicht auf die Mikropartikel aus Schritt a) einer Lösung oder Dispersion, die mindestens ein Polymer A enthält, das in den gastrointestinalen Flüssigkeiten unlöslich ist, in Mischung mit mindestens einem Polymer B, das einen Solubilisierungs-pH-Wert im pH-Bereich von 5 bis 7 aufweist,
c) Mischen der beschichteten Wirkstoff-Mikropartikel, die am Ende von Schritt b) erhalten werden, mit einem oder mehreren physiologisch akzeptablen Exzipienten, der/die eine Matrix bilden kann/können,
d) Agglomerieren der in Schritt c) gebildeten Mischung durch Komprimieren.

14. Verfahren nach dem vorhergehenden Anspruch, wobei die Polymere A und B wie in den Ansprüchen 1 bis 4 definiert sind.

15. Verfahren nach Anspruch 13 oder 14, wobei die am Ende von Schritt b) erhaltenen Mikropartikel wie in den Ansprüchen 5 bis 9 definiert sind.

16. Mikropartikel, die einen Kern aufweisen, der ganz oder teilweise aus mindestens einem Wirkstoff gebildet ist, wobei der Kern mit mindestens einer Schicht beschichtet ist, die einen dualen Freisetzungsmechanismus des Wirkstoffs bedingt, wobei der erste durch die Zeit bedingt ist und der zweite durch den pH-Wert bedingt ist,
wobei die Beschichtungsschicht aus einem Material gebildet ist, bestehend aus mindestens:
- 30 bis 50 %, bezogen auf das Gesamtgewicht der Beschichtung, mindestens eines Polymers A, das in gastrointestinalen Flüssigkeiten unlöslich ist und ausgewählt ist aus Ethylcellulose, Celluloseacetatbutyrat, einem Ammonium(meth)acrylat-Copolymer Typ "A" oder Typ "B", Poly(meth)acrylsäureestern und Mischungen davon, und
- 40 bis 60 Gew.-%, bezogen auf das Gesamtgewicht der Beschichtung, mindestens eines Polymers B, das einen Solubilisierungs-pH-Wert im pH-Bereich von 5 bis 7 aufweist und ausgewählt ist aus einem Methacrylsäure/Methylmethacrylat-Copolymer, einem Methacrylsäure/Ethylacrylat-Copolymer und Mischungen davon;
wobei die Beschichtungsschicht der Mikropartikel aus einer einzigen Schicht besteht, die durch das Material gebildet wird,
wobei die Mikropartikel den Wirkstoff gemäß einem modifizierten Freisetzungsprofil in drei Phasen freisetzen können, **gekennzeichnet durch**:
- bei Kontakt mit einem Medium, dessen pH-Wert einen Wert aufweist, der kleiner ist als der des Solublisierungs-pH-Werts des Polymers B, ein verzögertes und verlängertes Freisetzungsprofil mit einer gegebenen Latenzzeit, das im Bereich zwischen 0,5 und 12 Stunden, insbesondere zwischen 0,5 und 8 Stunden oder sogar zwischen 1 und 5 Stunden und gemäß einer halben Freisetzungszeit t_{1/2} im Bereich zwischen 0,75 und 24 Stunden, insbesondere zwischen 0,75 und 12 Stunden, oder sogar zwischen 0,75 und 8 Stunden, insbesondere zwischen 1 und 5 Stunden liegt, einer Zeit, an deren Ende die Hälfte des enthaltenen Wirkstoff freigesetzt ist;
- bei Kontakt mit einem Medium, dessen pH-Wert ein Wert ist, der größer ist als der des Solubilisierungs-pH-Werts des Polymers B, eine Freisetzung des Wirkstoffs ohne Latenzzeit und mit einem t_{1/2} im Bereich zwischen 0,1 und 2 Stunden;
wobei die Freisetzungskinetik des Wirkstoffs mit In-vitro-Auflösungstests bestimmt werden kann, die in einem USB-II-Gerät mit Quirlen mit einer Drehgeschwindigkeit der Quirle von 75 U/min, mittels UV-Spektrometrie einerseits in einem 0,1N-HCl-Medium und andererseits in einem 0,05M-Kaliumphosphat-Medium bei einem pH-Wert von 6,8 durchgeführt werden.

17. Mikropartikel nach dem vorhergehenden Anspruch, deren Beschichtung aus mindestens einem Polymer-B/Polymer-A-Paar gebildet ist, das aus den folgenden Paaren ausgewählt ist:
- Methacrylsäure/Ethylacrylat-Copolymer, 1:1/Ethylcellulose,
- Methacrylsäure/Methylmethacrylat-Copolymer 1:2/Ethylcellulose,
- Mischung aus Methacrylsäure/Ethylacrylat-Copolymer 1:1 und Methacrylsäure/Methylmethacrylat-Copolymer, 1:2/Ethylcellulose,
- Methacrylsäure/Ethylacrylat-Copolymer 1:1/Celluloseacetatbutyrat,
- Methacrylsäure/Methylmethacrylat-Copolymer 1:2/Celluloseacetatbutyrat,
- Mischung aus Methacrylsäure/Ethylacrylat-Copolymer 1:1 und Methacrylsäure/Methylmethacrylat-Copolymer 1:2/Celluloseacetatbutyrat,
- Methacrylsäure/Ethylacrylat-Copolymer 1:1/Ammonium(meth)acrylat-Copolymer Typ "A" oder Typ "B",
- Methacrylsäure/Methylmethacrylat-Copolymer 1:2/Ammonium(meth)acrylat-Copolymer Typ "A" oder Typ "B",
- Mischung aus Methacrylsäure/Ethylacrylat-Copolymer 1:1 und Methacrylsäure/Methylmethacrylat-Copolymer 1:2/Ammonium(meth)acrylat-Copolymer Typ "A" oder Typ "B".

## Claims

1. Tablet which is intended for the administration by the oral route of at least one active principle and which is capable of guaranteeing a dual release mechanism for the said active principle, the first being conditioned by the time and the second being conditioned by the pH,
in which the said active principle is present therein in the form of a microparticulate system, the microparticles of which have a core formed in all or part of the said active principle and coated with at least one layer which conditions the said release profile of said active principle and which is formed of a material composed of at least:
- from 30% to 50% by weight, with respect to the total weight of the said coating, of at least one polymer A which is insoluble in the gastrointestinal fluids and which is chosen from ethylcellulose, cellulose acetate butyrate, cellulose acetate, ammonio (meth)acrylate copolymers of type "A" or of type "B", esters of poly(meth)acrylic acids, and their blends;
- from 30% to 60% by weight, with respect to the total weight of the said coating, of at least one polymer B which has a pH value for dissolution within the pH range from 5 to 7 and which is chosen from:
∘ copolymer(s) of methacrylic acid and of methylmethacrylate,
∘ copolymer(s) of methacrylic acid and of ethyl acrylate,
∘ cellulose derivatives, such as cellulose acetate phthalate, cellulose acetate succinate, cellulose acetate trimellitate, hydroxypropylmethylcellulose phthalate or hydroxypropylmethylcellulose acetate succinate,
∘ shellac,
∘ polyvinyl acetate phthalate, and
∘ their blends, and
the said coating layer of the microparticles being composed of a single layer formed of the said material;
the said microparticles being dispersed within the tablet,
the said microparticles being capable of releasing the said active principle according to a three-phase modified release profile **characterized by**:
- in contact with an environment, the pH of which is at a lower value than that of the pH for dissolution of the polymer B, a delayed and prolonged release profile with a given latency time of between 0.5 and 12 hours, in particular between 0.5 and 8 hours, indeed even between 1 and 5 hours, and according to a time for half release t_{1/2} of between 0.75 and 24 hours, in particular between 0.75 and 12 hours, indeed even between 0.75 and 8 hours, especially between 1 and 5 hours, at the end of which time half of the active principle present is released;
- in contact with an environment, the pH of which is at a higher value than that of the pH for dissolution of the polymer B, a release of the active principle without a latency time and with a t_{1/2} of between 0.1 and 2 hours;
it being possible for the kinetics or release of the active principle to be determined by *in vitro* dissolution test carried out in a USP type II paddle device with a rotational speed of the paddles of 75 rpm, by UV spectrometry, on the one hand in a 0.1N HCl medium and, on the other hand, in a 0.05M potassium phosphate medium at pH 6.8.

2. Tablet according to the preceding claim, in which the coating of the microparticles comprises from 40% to 60% by weight of polymer (s) B, with respect to the total weight of the said coating.

3. Tablet according to either one of the preceding claims, in which the coating of the microparticles is formed of at least a mixture of ethylcellulose, of cellulose acetate butyrate or of ammonio (meth)-acrylate copolymer of type "A" or "B" with at least one copolymer of methacrylic acid and ethyl acrylate or one copolymer of methacrylic acid and of methylmethacrylate or one of their blends.

4. Tablet according to any one of the preceding claims, in which the coating comprises the polymers A and B in a polymer(s) B/polymer(s) A ratio by weight of greater than or equal to 0.75.

5. Tablet according to any one of the preceding claims, in which the coating of the microparticles additionally comprises at least one plasticizing agent.

6. Tablet according to the preceding claim, in which the coating of the microparticles comprises less than 25% by weight, in particular from 1% to 20% by weight and more preferably from 5% to 20% by weight of plasticizing agent(s), with respect to its total weight.

7. Tablet according to any one of the preceding claims, in which the coating positioned at the surface of the microparticles is present at a degree of coating varying from 3% to 85% by weight, in particular from 5% to 60% by weight, especially from 10% to 50% by weight, indeed even from 10% to 40% by weight and more particularly from 20% to 40% by weight of coating, with respect to the total weight of the said microparticle.

8. Tablet according to any one of the preceding claims, in which the coating positioned at the surface of the microparticles is obtained by spraying, in a fluidized air bed, a solution comprising at least the said polymers A and B in the solute state over particles of active principle(s).

9. Tablet according to any one of the preceding claims, in which the microparticles have an average diameter of less than or equal to 2000 µm, in particular of less than or equal to 1000 µm, especially of less than 800 µm, in particular of less than 600 µm, indeed even of less than 500 µm.

10. Tablet according to any one of the preceding claims, in which the active principle is chosen from anaesthetics, analgesics, antiasthmatics, agents for the treatment of allergies, anticancer agents, antiinflammatories, anticoagulants and antithrombotics, anticonvulsants, antiepileptics, antidiabetics, antiemetics, antiglaucomas, antihistaminics, antiinfectives, in particular antibiotics, antifungals, antivirals, antiparkinsonians, anticholinergics, antitussives, carbonic anhydrase inhibitors, cardiovascular agents, in particular hypolipidaemics, antiarrythmics, vasodilators, antianginals, antihypertensives, vasoprotectants and cholinesterase inhibitors, agents for the treatment of disorders of the central nervous system, stimulants of the central nervous system, contraceptives, fertility promoters, dopamine receptor agonists, agents for the treatment of endometriosis, agents for the treatment of gastrointestinal disorders, immunomodulators and immunosuppressants, agents for the treatment of memory disorders, antimigraines, muscle relaxants, nucleoside analogues, agents for the treatment of osteoporosis, parasympathomimetics, prostaglandins, psychotherapeutic agents, such as sedatives, hypnotics, tranquilizers, neuroleptics, anxiolytics, psychostimulants and antidepressants, agents for dermatological treatments, steroids and hormones, amphetamines, anorectics, nonanalgesic painkillers, antiepileptics, barbiturates, benzodiazepines, hypnotics, laxatives or psychotropics.

11. Tablet according to any one of the preceding claims, comprising at least two types of microparticles which differ from one another in distinct release profiles.

12. Tablet according to any one of the preceding claims, comprising at least two types of microparticles, in which said types differ from one another at least in the nature of the active principle which they comprise and/or in the composition of their coating and/or the thickness of their coating.

13. Process for the preparation of a tablet for the oral administration of at least one active principle according to any one of the preceding claims, comprising at least the stages consisting in:
a) having available microparticles formed in all or part of at least one active principle,
b) spraying, in a fluidized air bed, over the microparticles of stage a), a solution or dispersion comprising at least one polymer A which is insoluble in gastrointestinal fluids as a mixture with at least one polymer B having a pH value for dissolution within the pH range from 5 to 7,
c) mixing the coated microparticles formed of active principles, obtained on conclusion of stage b), with one or more physiologically acceptable excipients capable of forming a matrix,
d) agglomerating by compression the mixture formed in stage c).

14. Process according to the preceding claim, in which the polymers A and B are as defined in Claims 1 to 4.

15. Process according to Claim 13 or 14, in which the microparticles obtained on conclusion of stage b) are as defined in Claims 5 to 9.

16. Microparticles having a core formed in all or part of at least one active principle, the said core being coated with at least one layer conditioning a dual mechanism for release of the said active principle, the first being conditioned by the time and the second being conditioned by the pH,
the said coating layer being formed of a material composed of at least:
- from 30% to 50%, with respect to the total weight of the said coating, of at least one polymer A which is insoluble in gastrointestinal fluids and which is chosen from ethylcellulose, cellulose acetate butyrate, an ammonio (meth)acrylate copolymer of type "A" or of type "B", esters of poly (meth) acrylic acids and their blends, and
- from 40% to 60% by weight, with respect to the total weight of the said coating, of at least one polymer B which has a pH value for dissolution within the pH range varying from 5 to 7 and which is chosen from a copolymer of methacrylic acid and of methyl methacrylate, a copolymer of methacrylic acid and ethyl acrylate and their blends;
the said coating layer of the microparticles being composed of a single layer formed of the said material,
the said microparticles being capable of releasing the said active principle according to a three-phase modified release profile **characterized by**:
- in contact with an environment, the pH of which is at a lower value than that of the pH for dissolution of the polymer B, a delayed and prolonged release profile with a given latency time of between 0.5 and 12 hours, in particular between 0.5 and 8 hours, indeed even between 1 and 5 hours, and according to a time for half release t_{1/2} of between 0.75 and 24 hours, in particular between 0.75 and 12 hours, indeed even between 0.75 and 8 hours, especially between 1 and 5 hours, at the end of which time half of the active principle present is released;
- in contact with an environment, the pH of which is at a higher value than that of the pH for dissolution of the polymer B, a release of the active principle without a latency time and with a t_{1/2} of between 0.1 and 2 hours;
it being possible for the kinetics of release of the said active principle to be determined by *in vitro* dissolution tests carried out in a USP type II paddle device with a rotational speed of the paddles of 75 rpm, by UV spectrometry, on the one hand in a 0.1N HCl medium and, on the other hand, in a 0.05M potassium phosphate medium at pH 6.8.

17. Microparticles according to the preceding claim, the coating of which is formed of at least one polymer B/polymer A pair chosen from the following pairs:
- copolymer of methacrylic acid and ethyl acrylate (1:1)/ethylcellulose,
- copolymer of methacrylic acid and methyl methacrylate (1:2)/ethylcellulose,
- blend of copolymer of methacrylic acid and ethyl acrylate (1:1) and of copolymer of methacrylic acid and methyl methacrylate (1:2)/ethylcellulose,
- copolymer of methacrylic acid and ethyl acrylate (1:1)/cellulose acetate butyrate,
- copolymer of methacrylic acid and methyl methacrylate (1:2)/cellulose acetate butyrate,
- blend of copolymer of methacrylic acid and ethyl acrylate (1:1) and of copolymer of methacrylic acid and methyl methacrylate (1:2)/cellulose acetate butyrate,
- copolymer of methacrylic acid and ethyl acrylate (1:1)/ammonio (meth)acrylate copolymer of type "A" or type "B",
- copolymer of methacrylic acid and methyl methacrylate (1:2)/ammonio (meth)acrylate copolymer of type "A" or type "B",
- blend of copolymer of methacrylic acid and ethyl acrylate (1:1) and of copolymer of methacrylic acid and methyl methacrylate (1:2)/ammonio (meth)acrylate copolymer of type "A" or type "B".
